# EUROPEAN PATENT APPLICATION

(11) **EP 4 137 154 A1**
(43) Date of publication of application: **22.02.2023**
(21) Application number: 21788572.2
(22) Date of filing: 16.04.2021
(51) Int. Cl.: A61K 39/395, C07K 16/30, C07K 14/725, A61P 35/02

(54) **THERAPEUTIC TREATMENT FOR T-CELL ACUTE LYMPHOBLASTIC LEUKEMIAS USING A MONOCLONAL ANTIBODY AGAINST THE PRE-T CELL RECEPTOR**

(30) Priority: 17.04.2020 ES 202030309
(71) Applicant: Consejo Superior De Investigaciones Científicas, 28006 Madrid (ES); Fundación Uno Entre Cien Mil, 28003 Madrid (ES)
(72) Inventor: TORIBIO GARCÍA, María Luisa, 28049 Madrid (ES); ALARCÓN SÁNCHEZ, Balbino, 8049 Madrid 2 (ES); ALCAIN SÁNCHEZ, Juan, 28049 Madrid (ES); BAYÓN CALDERÓN, Fátima, 28049 Madrid (ES); FUENTES VILLAREJO, Patricia, 28049 Madrid (ES); GARCÍA PEYDRÓ, Marina, 28049 Madrid (ES); GONZÁLEZ GARCÍA, Sara, 28049 Madrid (ES); MURCIA CEBALLOS, Alba, 28049 Madrid (ES)
(74) Representative: Pons
(86) International application number: PCT/ES2021/070254
(87) International publication number: WO 2021/209670

(57) **Abstract**

The present invention relates to the treatment of T-cell acute lymphoblastic leukemia (T-ALLs) by means of immunotherapy with a monoclonal antibody which recognizes the pre-TCR receptor which, due to its presence in all phases of T-ALL pre-TCR+ development, can also be used as a leukemia-initiating cell (LIC) biomarker and as a therapeutic target useful for monitoring minimal residual disease and identifying compounds inhibiting the disease.

## Description

The present invention belongs to the chemistry and pharmacy sector, specifically it relates to the therapeutic use of agents for the specific recognition of human pre-TCR receptor for the treatment and/or prevention of human acute lymphoblastic leukemia (ALL) of the T-cells (T-ALL), including the treatment and/or prevention of relapses of said disease.

### BACKGROUND OF THE INVENTION

The most common cause of childhood morbidity is acute lymphoblastic leukemia (ALL), an aggressive tumor arising from oncogenic transformation of T or B lymphoid progenitors during their development (T-ALL and B-ALL leukemia, respectively), of which T-ALL constitutes 10-15% of the cases (Karrman and Johansson, Genes Chromosomes Cancer, 56 (2), 89-116, February 2017).

ALL, particularly T-ALL, has a poor prognosis, although the development of intensive chemotherapy treatments in recent decades has notably increased the life expectancy of patients with ALL of both cell types. The 5-year survival rate for T-ALL patients is over 70%. In cases of relapses, the survival rate of T-ALL patients drops drastically to as low as 20% (Karrman and Johansson, Genes Chromosomes Cancer, 56 (2), 89-116, February 2017).

Most ALL relapses occur in early post-treatment phases and reflect the treatment resistance of a clonal population of cells that were already present at diagnosis (Bailey et al., Lancet Oncol. 5 9: 873-883. 2008), and are responsible for the onset, spread, and persistence of leukemia, hence the name "leukemia-initiating cells" (LICs). The success of ALL treatment will therefore depend on the efficient elimination of LICs. Although significant efforts have been made to design new treatments that fulfill this objective, the development of specific anti-LIC drugs which reduce the frequency of relapses is still in its initial phases. This work is complex, since LICs represent less than 1% of the total leukemic population and specific markers for these cells have not yet been found. Accordingly, the success of any new therapy depends on the development of strategies which allow the LICs of T-ALL and B-ALL to be identified and characterized, so as to promote the design of new tactics allowing their elimination.

T-lymphocytes interact with antigens through the T-cell antigen receptor (TCR, for *T-Cell Receptor*) which recognizes its target antigen processed and presented in the form of peptides by major histocompatibility complex (MHC) molecules. This TCR receptor is a specific heterodimer of each T-cell clone which is expressed on the surface associated with the CD3 complex. About 90% of peripheral blood T-cells (Tαβ cells) express a TCR formed by a TCRα polypeptide chain and a TCRβ polypeptide chain. The remaining 10% (Tγδ cells) express a TCR formed by a TCRγ chain and a TCRδ chain. Each of these TCR chains is different in each T-cell clone and is made up of a unique combination of domains referred to as variable (V), [diversity (D)], binding (J), and constant (C) domains. In each T-cell clone, the combination of the V, (D), and J domains in both α and β chains or in both γ and δ chains participates in antigen recognition in a specific manner through 3 complementary regions (CDR for *Complementarity Determining Region*), mainly through CDR3.

Like immunoglobulin genes, TCR receptor genes consist of a series of regions encoding the V, D, J, and C domains that are rearranged during T-cell development in the thymus. Intrathymic progenitors of Tαβ lymphocytes have to go through at least two selection processes mediated by signals through two different receptors, the pre-T cell receptor or pre-TCR, and the mature receptor or TCRαβ, that are expressed sequentially during intrathymic development (Borst et al., Curr Opin Immunol 1996; 8: 181-90). In the thymus, genes encoding the TCRβ chain are rearranged and expressed before those corresponding to the TCRα locus. Thymocytes that productively rearrange the TCRβ locus first express a pre-TCR complex, independent of TCRα, made up of a TCRβ chain covalently linked to an invariant pre-TCRα chain (pTα), which associates with the CD3 complex and promotes a quality control process generally known as "*β-selection*"(Groettrup et al., Cell 1993; 75: 283-94). β selection induces the survival and proliferative expansion of those thymocytes in which correct TCRβ rearrangement takes place and simultaneously determines the inhibition of other rearrangements in this locus or allelic exclusion (Dudley et al., Immunity 1994; 1: 83-93). Cell expansion induced by pre-TCR ends abruptly after its internalization and degradation, and in the resulting population of resting thymocytes, rearrangements are then initiated in the TCRα locus (Petry et al., Exp Med 1993; 178: 615-22). Following the expression of TCRα and the substitution of pTα with TCRα, the TCRαβ receptor is expressed in association with CD3 on the cell membrane, and thymocytes undergo a second selection process known as "positive selection", are rescued from programmed cell death, and the final differentiation of these pre-T cells into mature Tαβ cells takes place. By means of an additional "negative selection" process, clonal elimination of those thymocytes with self-reactive TCRαβ receptors will be induced, thereby ensuring tolerance of the individual to the components themselves.

A human pTa-specific monoclonal antibody (mAb), referred to as K5G3, was developed utilizing the selective expression of the pTα molecule as a target for the identification of pre-T lymphocytes, said antibody being capable of specifically identifying T-ALL leukemia cells representative of the pre-T maturation stage, and therefore being useful for analyzing the capacity of such leukemias to respond to chemotherapy by means of analyzing residual cells in blood (ES2190880A1).

Currently, minimal residual disease (MRD) analysis by means of flow cytometry (for aberrant immunophenotypes), fusion gene detection by PCR (for leukemias with translocation in the TCR or others), or TCR rearrangement is the clearest and most powerful treatment response prognostic factor existing for T-ALL, both in children and adults. Having a specific leukemia marker is very useful for monitoring the patient's response to treatment and for the early identification of groups at high risk of relapse, with the possibility of adapting and even anticipating treatment in these patients.

A major advancement in the treatment of ALL has been the incorporation of targeted therapy by means of mAb, such as anti-CD20, anti-CD19, and anti-CD22 mAbs, among others. These mAbs are directed against malignant B-cells, but they also recognize normal B-cells that are eliminated along with tumor cells, therefore, it is necessary to infuse patients with immunoglobulins. In the case of T-ALL, there are some studies conducted with anti-CD52 mAb which recognizes both T- and B-cells, with not very encouraging results and considerable side effects due to the elimination of normal T-cells, which leads to the onset of a severe immunodeficiency (Angiolillo et al., Pediatr Blood Cancer. 2009; 53:978-83). More recently, other therapies such as T-cells with a chimeric antigen receptor (CAR-T), which are T-cells genetically modified to express a receptor which recognizes the tumor and are activated to eliminate same, have been developed. An example of such therapies is disclosed in US2020010555A1 which describes an anti-CD123 CAR-T immunotherapy treatment, with B-ALL being one of the treatment targets. Another example of immunotherapy targeting B-ALL and other B-cell leukemias is disclosed in document US2019062430 which refers specifically to CD19 CAR-T immunotherapy. These treatments improve the survival rate by 50 - 76% in cases of B-ALL relapses, but their efficacy in treating T-ALL has yet to be proven and appears doubtful at the moment because no specific targets of tumor T-cells, which prevent the elimination of normal T-cells and the fratricide of CAR-T, have been identified (Tandon and Punnett, Advances in Hematologic Malignancies, Gamal Abdul Hamid, IntechOpen, 23 October 2019).

In that sense, taking into account the current state of the art, the development of alternative therapeutic agents for the treatment of ALL, particularly T-ALL, which are directed against new therapeutic targets selectively involved in the disease, is crucial.

### DESCRIPTION OF THE INVENTION

The present invention relates to the identification of molecular targets in acute lymphoblastic leukemia (ALL) initiating cells (LICs), particularly T-cell ALL (T-ALL), and therapeutic agents against these targets.

In the present invention, the inventors demonstrate that a receptor which is expressed during the development of T-lymphocytes, known as pre-TCR (*pre-T cell receptor*), participates in the function of leukemia-initiating cells in a large group of childhood T-ALLs, and it is therefore a particularly relevant therapeutic target to act against the disease, particularly against relapses. In addition to identifying pre-TCR as a biomarker and potential therapeutic target present on the surface of T-ALL tumor cells responsible for relapses in patients, the inventors demonstrate the efficacy of a new immunotherapy strategy based on the administration of a monoclonal antibody (mAb) specific for said pre-TCR receptor in a preclinical model of human T-ALL xenotransplantation in mice.

In the present invention, the inventors demonstrate that the pre-TCR receptor is expressed on the surface of leukemic cells during all stages of disease progression in a human T-ALL generation model in mice. This is due to the fact that differentiation of T-lymphocytes in the maturation stage in which pre-TCR is expressed, expanding the pre-TCR+ population, is blocked. Furthermore, in the present invention, the inventors demonstrate that T-ALL pre-TCR+ cells exhibit leukemia-initiating (LIC) activity, and therefore have an important function in the progression and relapse of T-ALL in all patients, which indicates that pre-TCR is a suitable therapeutic target to approach the treatment of T-ALL and prevent relapses.

In that sense, one aspect of the present invention relates to the use of the human pre-TCR receptor, expressed on the surface of T-cells in acute lymphoblastic leukemias, as a therapeutic target for the identification, screening, or design of compounds, molecules, drugs, or the like, useful for the diagnosis, treatment, and/or prevention of leukemia, preferably T-cell acute lymphoblastic leukemia (T-ALL), more preferably T-ALL pre-TCR+, or useful for the diagnosis, treatment, and/or prevention of relapses of leukemia, preferably T-cell acute lymphoblastic leukemia (T-ALL), more preferably T-ALL pre-TCR+.

The term "therapeutic target" refers to the human pre-TCR receptor at the gene, protein, or mRNA level, which is useful for studying the biochemical effect of molecules capable of binding thereto and neutralizing its function or activity, and/or inducing cell lysis. The compounds, molecules, drugs, or the like, of interest will be those capable of exerting a blocking or neutralizing effect on said receptor preventing its gene or protein activity and/or inducing cell lysis. These molecules can be, for example, antibodies, antigen-binding fragments of antibodies, aptamers, interfering RNAs, small interfering RNAs (siRNAs), shRNAs, microRNAs (miRNAs), antisense oligonucleotides, peptides, peptidomimetics, small molecules, organic or inorganic chemical agents, etc.

Another aspect of the invention relates to an inhibitor of the human pre-TCR receptor, expressed on the surface of T-cells in acute lymphoblastic leukemias, for use as a medicament, preferably for use in the treatment and/or prevention of leukemia, more preferably T-cell acute lymphoblastic leukemia (T-ALL), even more preferably T-ALL pre-TCR+; or for use in the treatment and/or prevention of relapses of leukemia, more preferably T-cell acute lymphoblastic leukemia (T-ALL), even more preferably T-ALL pre-TCR+. In another preferred embodiment, said inhibitor is the antibody of the invention described below.

"Inhibitor of the human pre-TCR receptor" refers to an inhibitor of the pre-TCR protein or gene and can be any molecule which reduces the levels and/or activity of said receptor at the gene, protein, or mRNA level. Examples of such inhibitors are antisense sequences, preferably antisense mRNAs, siRNAs, shRNAs, human anti-pre-TCR antibodies or immunologically active fragments thereof, agonists, soluble forms of the human pre-TCR receptor which act as competitors, or molecules or compounds discovered or designed by means of screening methods.

Another aspect of the invention relates to the use of the human pre-TCR receptor as a biomarker, hereinafter, the biomarker of the invention, of acute lymphoblastic leukemia-initiating cells (LICs). In a preferred embodiment, the biomarker of the invention corresponds to the pTα subunit of the pre-TCR receptor. In another preferred embodiment, the acute lymphoblastic leukemia-initiating cells are leukemia-initiating T cells, more preferably T-ALL pre-TCR+ leukemia-initiating T cells. In that sense, the present invention proposes the use of the biomarker of the invention for the *in vitro* diagnosis, in a biological sample isolated *ex vivo*, of acute lymphoblastic leukemia, preferably T-cell acute lymphoblastic leukemia, and/or for the diagnosis and/or prognosis of relapses of acute lymphoblastic leukemia, preferably T-cell acute lymphoblastic leukemia.

The term "biomarker" as it is used in the present invention refers to a substance, the presence of which can be objectively determined and quantified, which is used as an indicator of the presence/absence of the disease or for its prognosis, particularly with respect to the possibility of relapses. The biomarker of the invention can be detected and/or quantified, that is, its presence can be detected or changes in the amount or concentration thereof can be detected and/or quantified.

The term "leukemia", as it is used herein, refers to the accepted medical term for a cancer of the blood-forming tissues of the bone marrow. It is characterized by the growth of immature white blood cells. The disease can be classified based on which blood cells are abnormal. Lymphomas are cancers of the lymphoid tissue (lymph nodes, spleen, and other organs of the immune system).

The term "acute lymphoblastic leukemia (ALL)", as it is used herein, refers to a hematologic cancer characterized by oncogenic transformation of lymphoid progenitors during their development.

The expression "T-cell acute lymphoblastic leukemia", as it is used herein, refers to a hematologic cancer characterized by abnormal growth of T-cell progenitors (a type of white blood cell - leukocytes) which originate in the thymus and progress rapidly. The expression "pre-TCR+ leukemia-initiating cells", as it is used herein, refers to leukemia-initiating T-cells which express the pre-TCR receptor on their cell surface.

Another aspect of the invention relates to an *in vitro* method, hereinafter the method of the invention, for monitoring minimal residual disease (MRD) in ALL patients, preferably T-ALL patients, which comprises detecting and/or quantifying the biomarker of the invention in an isolated biological sample obtained from the patient, wherein the detection of the biomarker in the isolated biological sample is indicative of the presence of T-cell acute lymphoblastic leukemia-initiating cells (LICs), and therefore of the existence of minimal residual disease in the patient and of a prognosis associated with relapse.

The term "monitor", as it is used herein, refers to observing and checking the progress of the disease evolution over a time period.

The expression "monitoring minimal residual disease (MRD)", refers to the detection and possible quantification of the amount of residual tumor cells in patients, preferably leukemia-initiating cells (LICs), beyond the sensitivity of cytomorphology. In that sense, monitoring MRD can be defined as those methods which enable the detection of at least one tumor cell out of 10,000 cells. Possible methods for monitoring MRD include, without limitation, cytogenetics, flow cytometry and its variants, such as fluorescence-activated cell sorting (FACS), or multiparametric flow cytometry of immunophenotypes associated with leukemia, polymerase chain reaction (PCR) amplification techniques, high-throughput sequencing techniques, next-generation flow cytometry, digital droplet PCR, and next-generation sequencing.

Obtaining an isolated biological sample from the patient includes any procedure which allows obtaining from the subject a sample which can be analyzed by means of the method of the invention. In a preferred embodiment of the method of the invention, the biological sample is selected from the list consisting of: peripheral blood and bone marrow sample. In a more preferred embodiment, the isolated biological sample is a bone marrow sample.

The expression "detecting and/or quantifying the biomarker of the invention in the isolated biological sample" refers to any procedure which enables identifying the presence and/or measuring the amount of the biomarker of the invention in the sample. Applicable detection and/or quantification methods are, for example, the same as those used to determine MRD, or Western blot, NMR, flow cytometry, immunoprecipitation assays, arrays, preferably antibody-based arrays, immunofluorescence, immunohistochemistry, enzyme-linked immunosorbent assays (ELISA), or any other enzymatic method, incubation with a specific ligand, chromatographic techniques preferably combined with mass spectrometry, spectroscopy, mass spectrometry, colorimetry, electrophoresis, or isoelectric focusing. Preferably, the detection and/or quantification of the biomarker of the invention is performed by means of *in situ* immunological, more preferably by means of ELISA-type immunoassay.

In another preferred embodiment, the detection and/or quantification of the biomarker of the invention in a biological sample isolated in the method of the invention is performed with a monoclonal antibody produced by means of the hybridoma deposited in the European Collection of Cell Cultures, Centre for Applied Microbiology and Research, Porton Down, Salisbury, Wiltshire, SP4 OJG, United Kingdom, on 8 August 2001, under accession number 01080805, capable of specifically recognizing the TCRβ- and CD3-associated pTα chain of the pre-T cell receptor (pre-TCR), hereinafter the "antibody of the invention" or K5G3 antibody. In a more preferred embodiment, the antibody of the invention is conjugated to a detectable marker.

Said "conjugation" can be carried out by means of any method known in the art to functionally connect the domains of an antibody with a marker producing a signal which can be visually detected including, without limitation, the recombinant fusion with or without intermediate domains, antibody-mediated fusion, non-covalent association and covalent bonding, for example, the binding of the antibody to the disulfide, hydrogen bonding, electrostatic bonding, and conformational bonding, for example, biotin-avidin associations. Conjugation with a label can be by chemical or recombinant means. Chemical means refers to a reaction between the antibody and the label or marker such that a covalent bond is formed between the two molecules in order to form a single molecule.

The term "detectable marker or label", as it is used herein, refers to molecules that can generate a signal which is detectable by means of a method suitable for said detection. Marker detection methods include, among others, fluorescence, light, confocal. and electron microscopy, magnetic resonance and spectroscopy; fluoroscopy, computerized tomography and positron emission tomography, enzyme-linked immunosorbent assays, size exclusion chromatography by fluorescence detection, lateral flow test, radioimmunoassay, radiolabeling, Western blot, immunohistochemistry, immunofluorescence, immunocytochemistry, flow cytometry, fluorescence-activated cell sorting, immunoprecipitation, and enzyme-type immunospot. Suitable markers include, among others, fluorescein, rhodamine, eosin and other fluorophores, radioisotopes, gold, gadolinium and other lanthanides, paramagnetic iron, fluorine-18 and other positron-emitting radionuclides. Furthermore, the probes or labels may be bi- or multifunctional and can be detected by means of more than one of the listed methods. The marker or label can be, for example, a chromogenic, fluorogenic, magnetic, electrodense, radioactive, and/or chemiluminescent compound. In a preferred embodiment of the present invention, the detectable label or marker is selected from the list consisting of: fluorescein isothiocyanate (FITC), allophycocyanin (APC), or any other relevant fluorescent dye in flow cytometry, biotin, horseradish peroxidase (HRP), gold beads, and paramagnetic iron.

In another aspect, the present invention relates to the antibody of the invention, for use as a medicament. The antibody of the invention targets the pTα chain of the pre-TCR, which represents an additional value for use thereof as a medicinal product, since the pTα chain is selectively expressed during intrathymic development but is absent in peripheral T-lymphocytes, which thereby allows preventing adverse effects due to the elimination of normal T-cells which could induce an aggressive immunodeficiency.

In the present invention, the term "antibody" refers to immunoglobulin molecules or immunologically active portions of immunoglobulin molecules, that is, molecules containing a binding site for antigens that bind specifically (immune reactions). Examples of immunologically active portions of immunoglobulin molecules include F(ab) and F(ab')2 fragments, which can be generated by treating the antibody in a recombinant manner or with an enzyme such as pepsin.

The expression "monoclonal antibody" refers to a population of antibody molecules containing a single type of antigen-binding site capable of immunologically reacting with a particular epitope of the antigen. The monoclonal antibody can be biologically altered by means of genetic manipulation, or it can be synthetic. Furthermore, the antibody may not have, in whole or in part, portions which are required for recognizing the antigen (human pre-TCR receptor), with such parts being replaced by others that impart additional advantageous properties to the antibody.

Methods for producing and detecting specific antibodies by means of hybridoma technology are routine and well known in the art. Briefly, mice can be immunized with a non-murine antigen and once an immune response is detected, for example, once antibodies specific for the antigen are detected in the mouse serum, the spleen of the mouse is harvested and splenocytes are isolated. Next, the splenocytes are fused with any suitable myeloma cell by means of well-known techniques. Hybridomas are selected and cloned by means of limited dilution. The hybridoma clones are then assayed by means of methods known in the art for cells which secrete antibodies capable of binding to pre-TCR. Ascites fluid, which generally contains high levels of antibodies, can be generated by immunizing mice with positive hybridoma clones.

The antibody of the invention can also be a recombinant antibody, chimeric antibody, humanized antibody, bispecific antibody, synthetic antibody, or a combination of any of the above.

A "chimeric" antibody is formed by the region of the H (heavy) chain and/or L (light) chain which correspond to the antibody sequences of certain species or belong to a class or subclass of certain antibodies, whereas the remaining parts are identical or homologous to the corresponding sequences in antibodies derived from other species or belonging to other classes or subclasses of antibodies, and to fragments of such antibodies, such that it exhibits the desired biological activity. A "humanized" antibody is a chimeric antibody in which the region recognizing the antigen comes from another non-human organism and the remaining regions of the antibody correspond to human antibody sequences. In a preferred embodiment, the antibody of the invention is humanized.

The examples disclosed in the present invention suggest that the antibody of the invention also induces the internalization of pre-TCR from the cell surface, which is a molecular mechanism that can be exploited from a therapeutic viewpoint, since therapies based on the use of monoclonal antibodies attached to toxins are particularly efficient in cases of internalization of the corresponding molecular targets. In that sense, in another preferred embodiment, the antibody of the invention is coupled to a toxin.

The term "coupled", as it is used in the present invention, refers to the fact that the antibody is functionally connected or conjugated to the toxin, including without limitation, the recombinant fusion with or without intermediate domains, antibody-mediated fusion, non-covalent association and covalent bonding, for example, the binding of the antibody to the disulfide, hydrogen bonding, electrostatic bonding, and conformational bonding, for example, biotin-avidin associations. Coupling with a toxin can be by chemical or recombinant means. Chemical means refers to a reaction between the antibody and the toxin such that a covalent bond is formed between the two molecules in order to form a single molecule. Alternatively, the antibody and the toxin can be conjugated by means of a linker or connector.

The toxin coupled to the antibody can be selected, without limitations, from the list consisting of: cytotoxins or cytostatic agents, radioactive or radiolabeled molecules, chemotherapeutic drugs and agents, or any combination thereof.

The term "medicament", as it is used herein, refers to any substance or combination of substances which has properties for the treatment or prevention of diseases in organisms, preferably in human beings, or which may be used or administered to organisms, preferably to human beings, in order to restore, correct, or modify physiological functions by exerting a pharmacological, immunological, or metabolic effect. The use of the antibody of the invention as a medicament can be carried out alone with the antibody or in combination with other medicaments or compositions by way of a combined therapy, and can be administered simultaneously or at different time intervals (sequentially).

In another preferred embodiment, the antibody of the invention is used in the diagnosis, treatment, and/or prevention of leukemia, preferably T-cell acute lymphoblastic leukemia (T-ALL), more preferably T-ALL pre-TCR+.

The term "treatment", as it is used herein, refers to combating the effects caused as a result of the disease or pathological condition of interest in a subject (preferably a mammal, and more preferably a human), including:
i) Inhibiting the disease or pathological condition, that is, stopping its development;
ii) Relieving the disease or pathological state, that is, causing the regression of the disease or the pathological state or its symptomatology;
iii) Stabilizing the disease or pathological state.

The term "prevention", as it is used herein, consists of preventing the onset or reapperance of the disease, that is, preventing the disease or disease state or relapse of the disease in a subject (preferably a mammal, and more preferably a human), particularly, when said subject is predisposed to the pathological condition but has not yet been diagnosed.

In another preferred embodiment, the antibody of the invention is used in the diagnosis, treatment, and/or prevention of relapses of leukemia, preferably relapses of T-cell acute lymphoblastic leukemia (T-ALL), more preferably relapses of T-ALL pre-TCR+.

The term "relapse", as it is used herein, refers to leukemia which had shown a regression or plateau after treatment, resumes its development or progression, or develops metastases.

Another aspect of the invention relates to a pharmaceutical composition, hereinafter the pharmaceutical composition of the invention, comprising the monoclonal antibody of the invention, obtained by the hybridoma deposited in the European Collection of Cell Cultures under accession number 01080805, capable of specifically recognizing the TCRβ- and CD3-associated pTα chain of the pre-T cell receptor (pre-TCR), for use thereof in the treatment and/or prevention of leukemia, preferably T-cell acute lymphoblastic leukemia (T-ALL), more preferably T-ALL pre-TCR+.

In a preferred embodiment, the pharmaceutical composition of the invention is used in the treatment and/or prevention of relapses of leukemia, preferably relapses of T-cell acute lymphoblastic leukemia (T-ALL), more preferably relapses of T-ALL pre-TCR+.

In another preferred embodiment of the pharmaceutical composition of the invention, the antibody is coupled to a toxin.

In another preferred embodiment of the pharmaceutical composition of the invention, the antibody is humanized.

In another preferred embodiment of the invention, the pharmaceutical composition of the invention further comprises an acceptable pharmaceutical adjuvant, an acceptable pharmaceutical carrier, and/or another active ingredient.

The term "acceptable pharmaceutical adjuvant", as it is used herein, refers to a substance which aids the absorption of the elements of the composition of the invention, stabilizes said elements, activates or aids the preparation of the composition in the sense of giving it consistency or providing flavors that make it more pleasant. In that sense, excipients or adjuvants could have the function of holding the ingredients together such as, for example, in the case of starches, sugars or celluloses, a sweetening function, a coloring function, the function of protecting the composition, such as, for example, to isolate it from the air and/or humidity, the function of filling a tablet, capsule, or any other form of presentation, the function of disintegrating in order to facilitate the dissolution of components and the absorption thereof in the intestine, without excluding other types of excipients not mentioned in this paragraph.

The term "acceptable pharmaceutical carrier", as it is used herein, refers to a substance used in the composition to dilute any of the components forming the composition up to a certain volume or weight. The pharmaceutically acceptable carrier is a substance which is inert or exhibits an action identical to any of the elements comprised in the composition of the present invention. The function of the carrier is to facilitate the incorporation of other elements, allow better dosing and administration or give the composition consistency and shape.

The composition of the present invention can also take the form of a sustained release drug formulation or any other conventional release system, such as, for example, nanoparticles, liposomes or nanospheres, polymeric material, biodegradable or non-biodegradable implant, or biodegradable microparticles, such as, for example, biodegradable microspheres. Said composition and/or its formulations can be administered to an animal, and therefore to human beings, in different ways, inter alia, intraperitoneal, intravenous, intradermal, intraspinal, intrastromal, intra-articular and intrasinovial, intrathecal, intralesional, intra-arterial, intramuscular, intranasal, intracraneal, subcutaneous, intraorbital, intracapsular, topical, by means of transdermal patches, percutaneously, spray nasal, surgical implant, internal surgical painting, or infusion pump.

The administration of the antibody of the invention or of the pharmaceutical composition of the invention will be carried out in a therapeutically effective dose which is sufficient to demonstrate a benefit for the patient, preferably in relation to a decrease in the number of LICs, more preferably T-ALL LICs, or in the replicative capacity of LICs in the patient. Such benefit may involve improvement of at least one symptom related to acute lymphoblastic leukemia. Prescription of the treatment, that is, decisions about dosages, frequency, duration, etc., will fall under the responsibility of the general practitioner or specialist who cares for the affected patient.

The term "therapeutically effective dose" or "therapeutically effective amount", as it is used in the present invention, refers to an amount (or amount per unit mass of the individual to whom the dose is administered) of a drug or therapeutic agent which causes a detectable therapeutic effect in an individual or a group of individuals to whom the dose is administered, causing minimal side or toxic effects. The therapeutically effective amount useful for producing a therapeutic effect can be determined by means of conventional techniques well known to those skilled in the art. The term "therapeutically effective dose-50" or "therapeutically effective dose-95" includes a statistical value in which the therapeutic effect must be detectable in 50% or 95% of the individuals to whom the dose is administered. With respect to the toxic effects of the drug or compound, it is preferable that the effective therapeutic dose does not cause any toxic effect. However, although toxic effects may occur in some cases, a compromise may be reached in which these effects are considered to be preferable to the normal development of the disease or sickness without administration of the drug or compound, and can in turn be treated by means of additional therapies.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### DESCRIPTION OF THE FIGURES

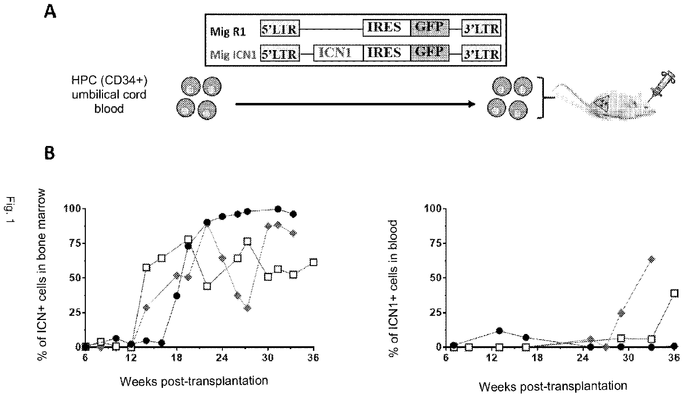

**Figure 1****. Generation of human leukemia T-ALL in NSG (NOD scid gamma) mice transplanted with umbilical cord blood hematopoietic progenitor cells (HPCs) transduced with active Notch1 oncogene (ICN1).** A) Diagram of the NSG mouse transduction and transplantation assay. B) Reconstitution kinetics of bone marrow (left) or peripheral blood (right) of different mice transplanted with CD34+ HPC cells transduced with ICN1.

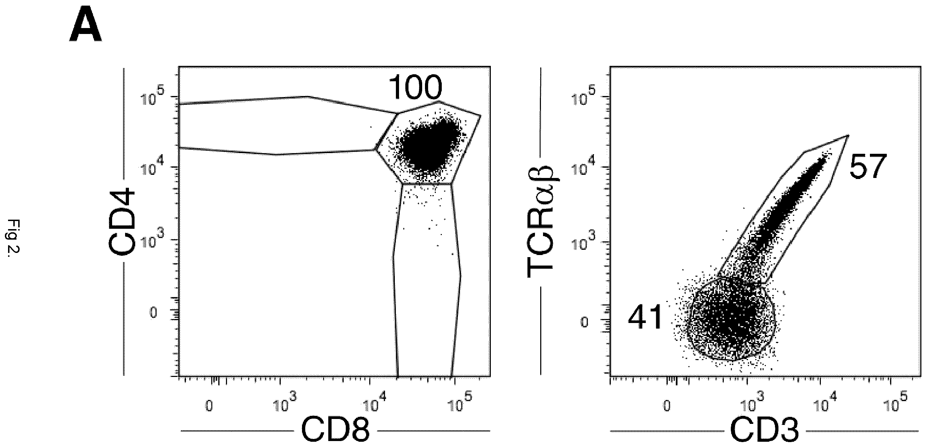

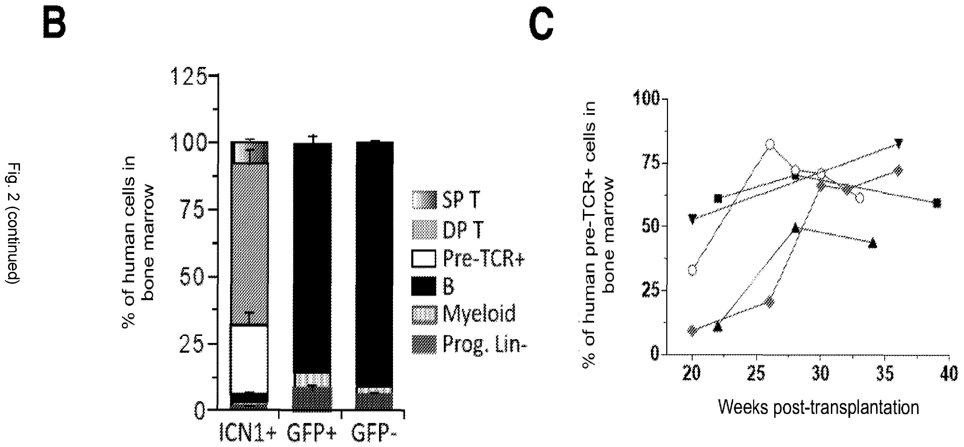

**Figure 2****. Analysis of pre-TCR expression in human T-ALL cells generated *de novo* after the expression of the ICN1 oncogene.** A) Phenotype of ICN1+ leukemic cells for the expression of CD4, CD8, CD3, and TCRαβ in the bone marrow of transplanted mice (Figure 1A) at 20 weeks post-transplantation. More than 45% of the ICN1+ CD4+CD8+ double-positive (DP) cells in the bone marrow express low levels of CD3 in the absence of TCRαβ, pre-TCR+ cell phenotype. B) The expression of pre-TCR is specific for ICN1+ leukemic cells, and not observed in ICN1- control cells. C) Pre-TCR+ leukemic cells are progressively expanded *in vivo* in animals with T-ALL until reaching >85% at 35-40 weeks post-transplantation.

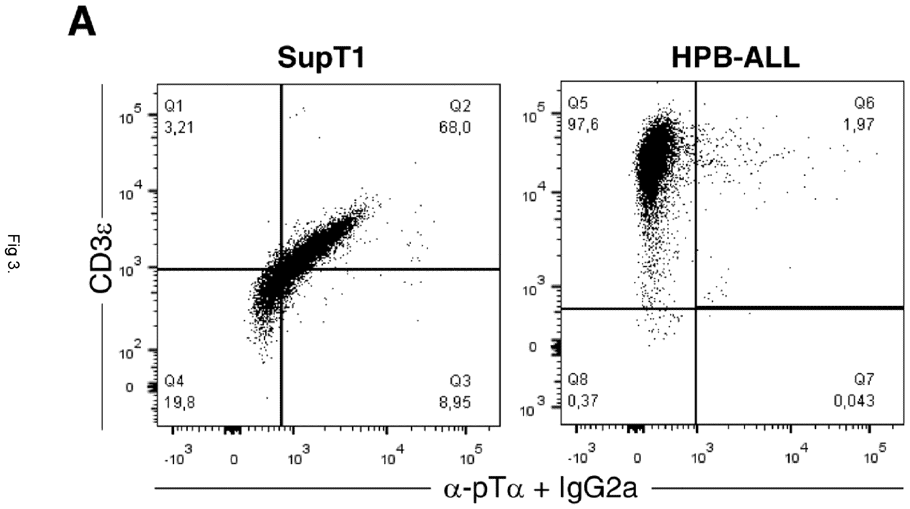

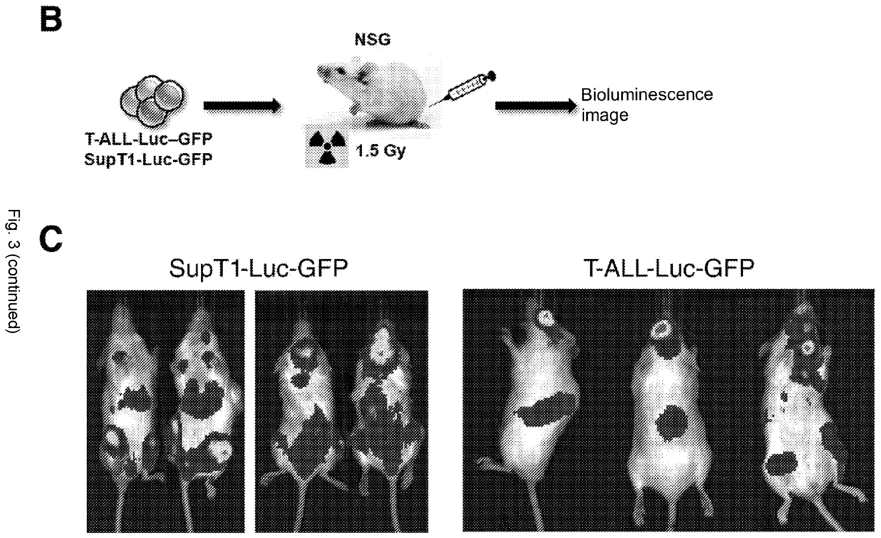

**Figure 3****. *In vivo* monitoring of T-ALL pre-TCR+ leukemia tumor progression in a preclinical model of xenotransplantation.** A) Specific expression of pre-TCR in the T-ALL SUPT1 line, and absence thereof in the T-ALL HPB-ALL line, defined by the simultaneous binding of anti-CD3ε and anti-pTα antibodies (UCHT1 and the antibody of the invention K5G3, respectively). B) Diagram of the xenotransplantation in NSG mice of primary T-ALL cells or SUPT1 cells transduced with luciferase for *in vivo* monitoring. C) Bioluminescence analysis of tumor progression in primary T-ALL or SUPT1 cells, as a pre-TCR+ leukemia/lymphoma model.

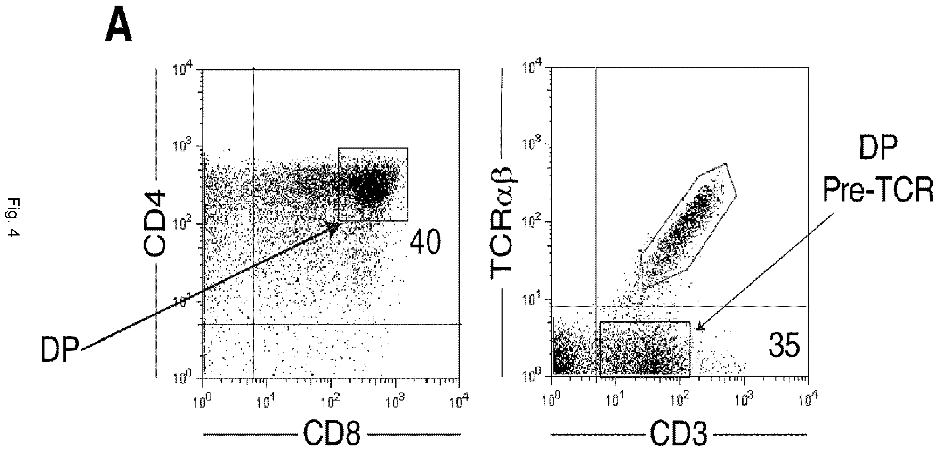

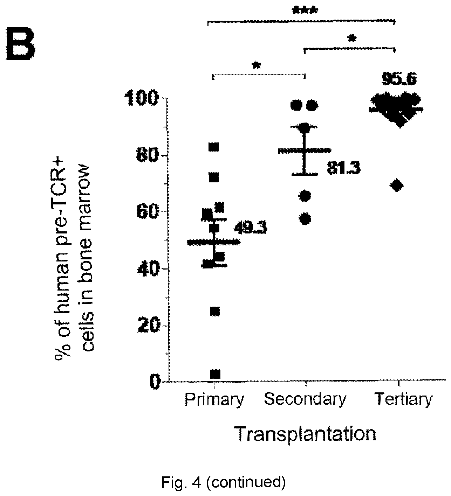

**Figure 4****. *In vivo* progression in the serial transplantations of T-ALL pre-TCR+ cells generated** ***de novo.*** (A) Heterogeneous TCRαβ+ and pre-TCR+ phenotype of leukemic cells generated *in vivo* from human ICN1+ HPC progenitors in the T-ALL pathogenesis model (Figure 1). (B) Enrichment (>95%) of pre-TCR+ leukemic cells generated in (A) serial transplantations in NSG mice.

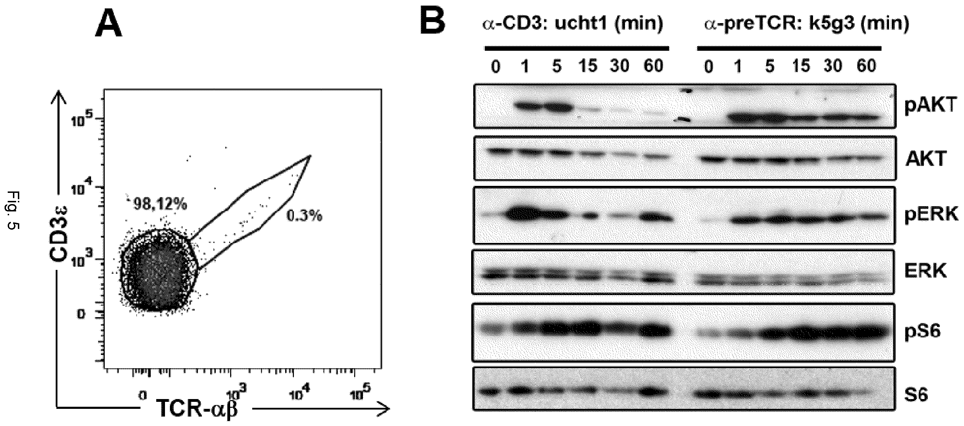

**Figure 5****. Study of the molecular cell survival and proliferation pathways activated by the pre-TCR expressed in cells with leukemia-initiating capacity (LICs) of T-ALL generated *de novo.*** A) 100% pre-TCR+ phenotype of T-ALL cells obtained from mouse bone marrow after 4 serial transplantations of a T-ALL generated *de novo* as described in Figure 1. B) *Western blot* analysis of the activation of the indicated survival and proliferation pathways, after stimulation of T-ALL pre-TCR+ cells in (A) by cross-linking with antibodies recognizing the CD3ε subunit or the pTα chain of the pre-TCR (UCHT1 and the antibody of the invention K5G3, respectively).

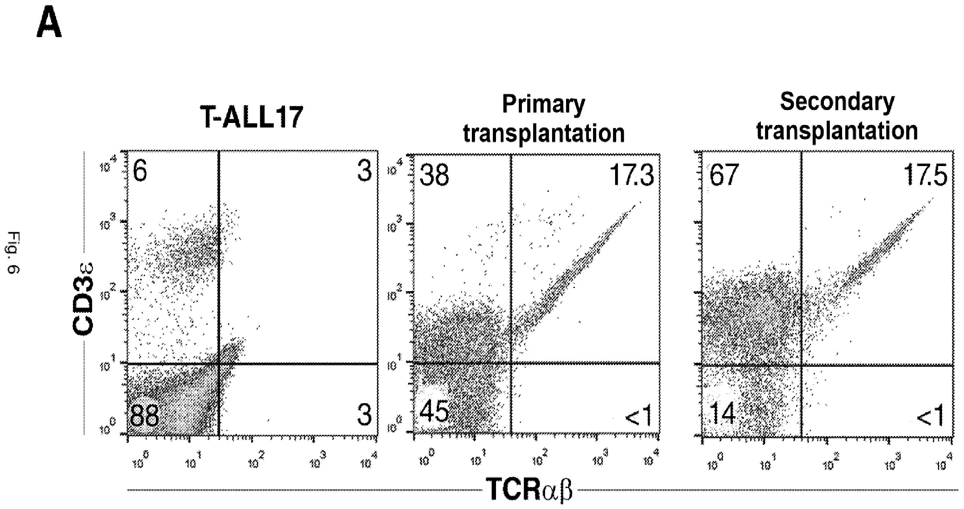

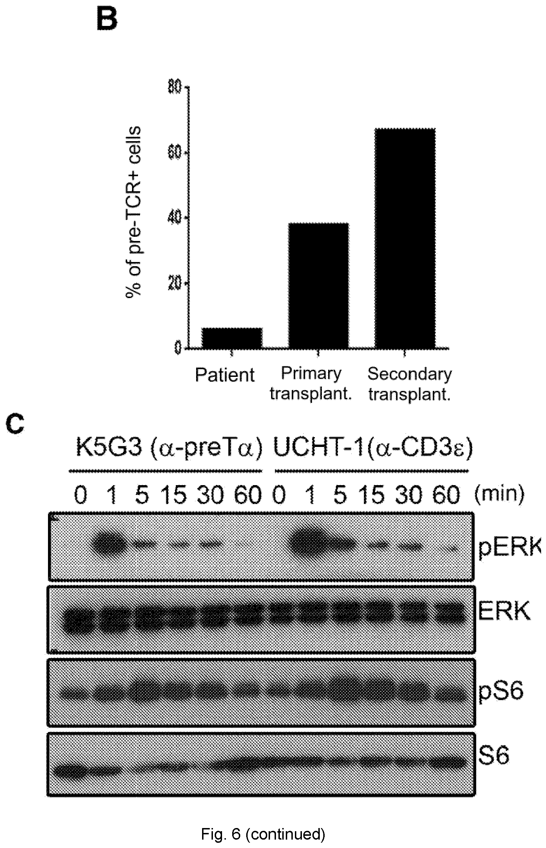

**Figure 6****. Analysis of *in vivo* progression in serial transplantations of primary T-ALL cells from patients and study of the function of their pre-TCR.** (A) Enrichment in pre-TCR+ leukemic cells from a patient in serial transplantations in NSG mice. (B) Enrichment quantification (>65%). C) Activation of molecular survival and proliferation pathways by cross-linking of the pre-TCR with antibodies recognizing the CD3ε subunit or the pTα chain of the pre-TCR (UCHT1 and the antibody of the invention K5G3, respectively) in primary T-ALL cells.

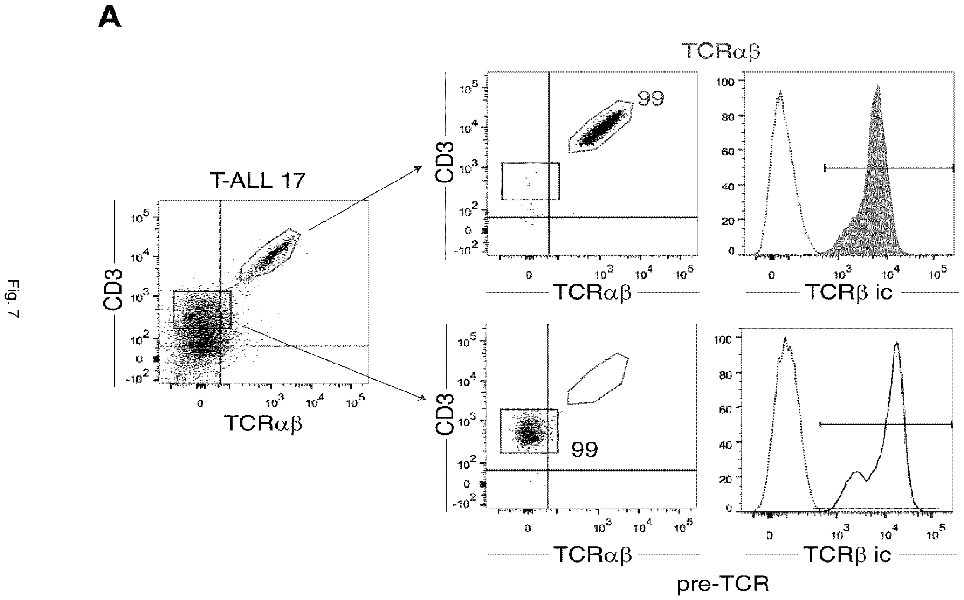

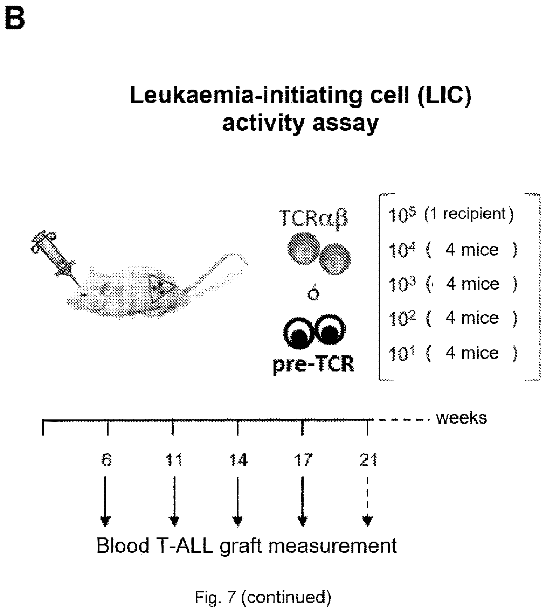

**Figure 7****. Separation of pre-TCR+ and pre-TCR- subpopulations of primary T-ALL cells and diagram of the *in vivo* assay of their LIC activity.** A) Primary T-ALL17 cells isolated from a patient's blood were separated by flow cytometry in two populations: CD3+TCRαβ- (pre-TCR+) and CD3+TCRαβ+ (pre-TCR-), both with intracellular expression of TCRβ (TCRβic). B) The isolated pre-TCR- and pre-TCR+ populations were transplanted in increasing numbers (10-10⁵) in NSG mice in which the graft in the blood was analyzed on the indicated days.

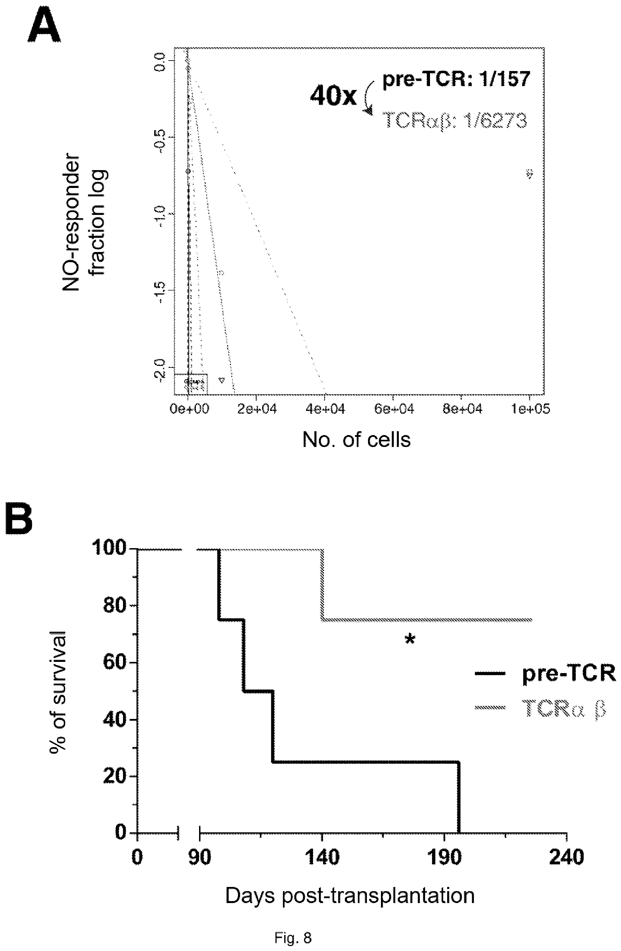

**Figure 8****. LIC activity of pre-TCR+ and pre-TCR- cells of a primary T-ALL leukemia in a patient.** A) Frequency of cells with LIC activity in the T-ALL pre-TCR+ and pre-TCR-subpopulations from the same patient. B) Survival of mice transplanted with the T-ALL pre-TCR+ and pre-TCR- populations.

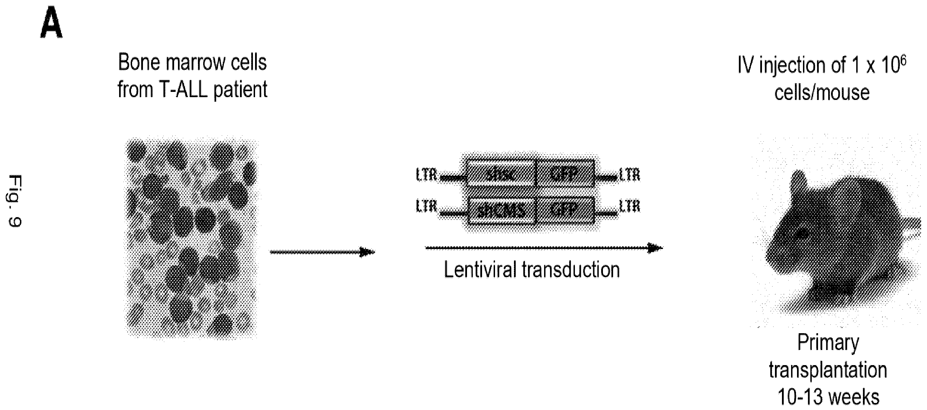

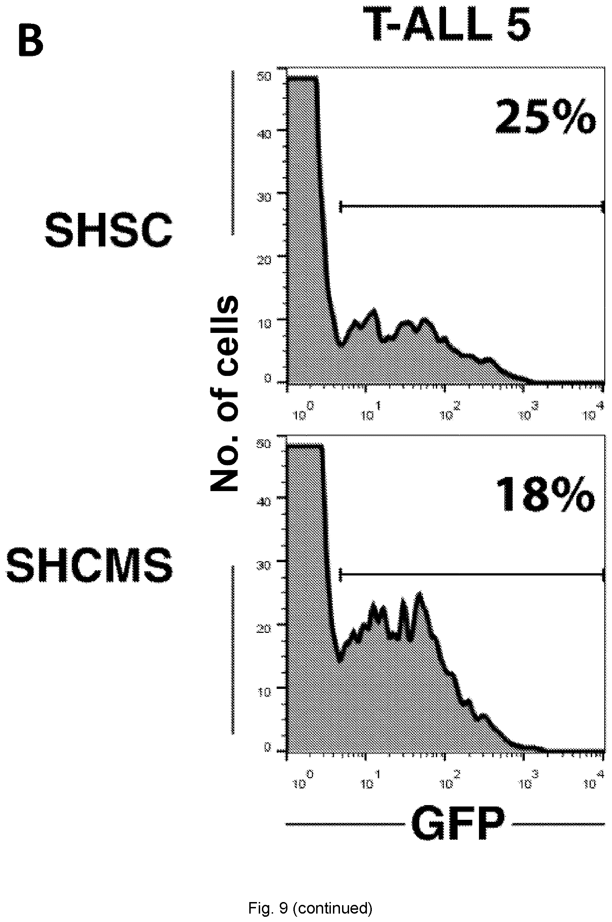

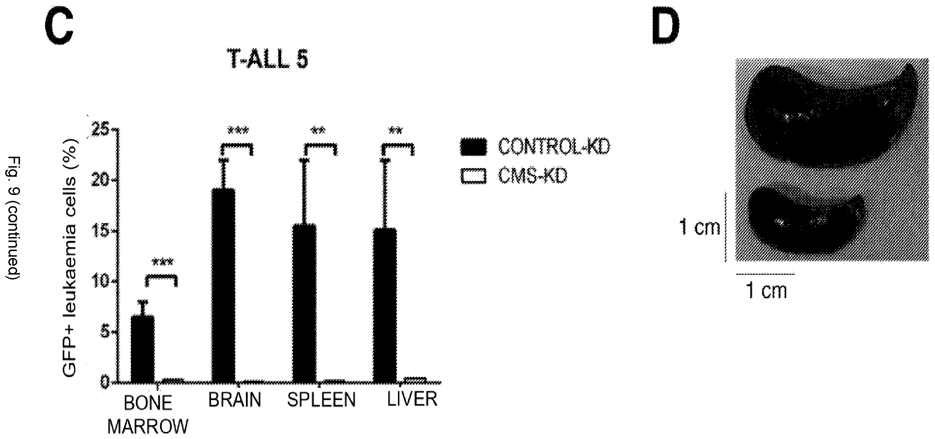

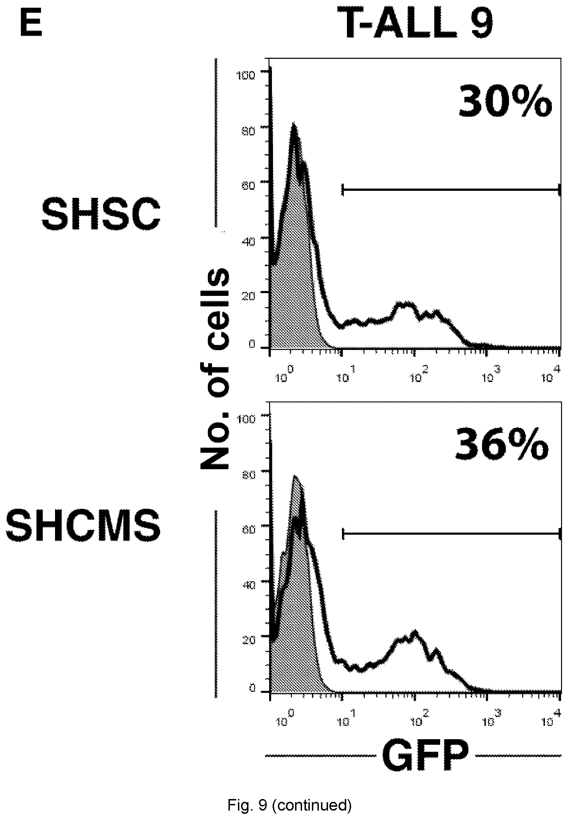

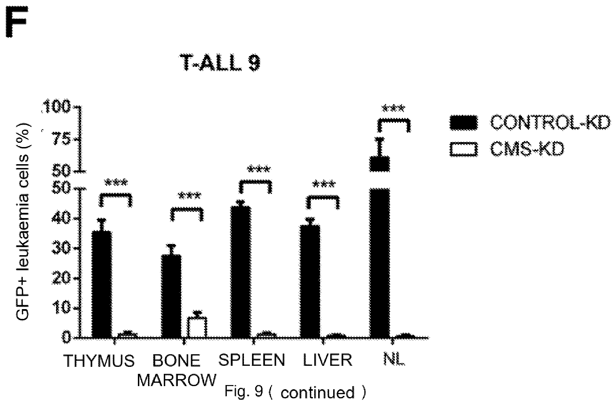

**Figure 9****. Analysis of the *in vivo* progression of a primary T-ALL transplanted after the functional inhibition of the pre-TCR.** A) Strategy for inhibiting the function of the pre-TCR expressed in T-ALL cells of patients by means of silencing the CMS adapter with shRNA-CMS lentiviral vectors. (B, E) Transduction efficiency measured by GFP expression. (C, F) Tumor progression in the indicated organs of T-ALL cells transduced with shRNA-CMS or shRNA-control (shsc) transplanted in Rag2-/- x yc-/- immunodeficient mice. (D) Silencing of CMS prevents tumor progression and splenomegaly induced by T-ALL cells.

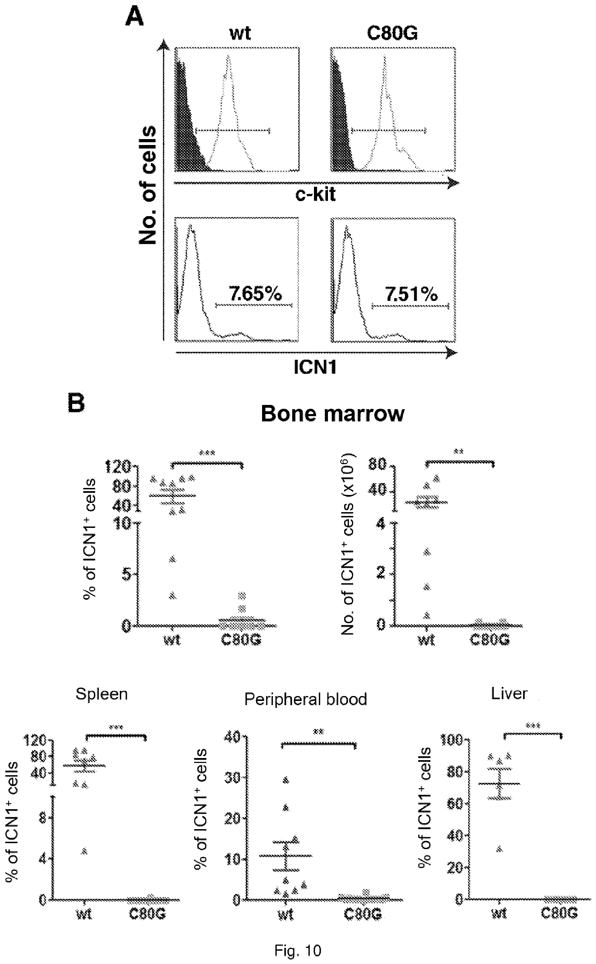

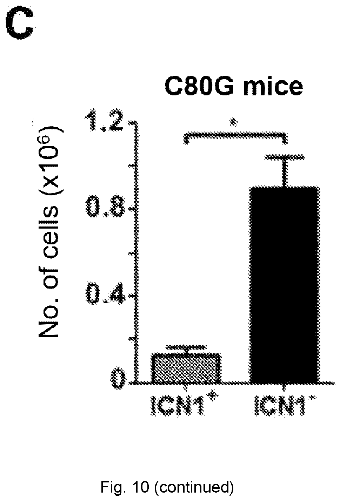

**Figure 10****. Analysis of the generation of T-ALL leukemia induced by the Notch1 oncogene in hematopoietic progenitors with a mutation affecting the function of the pre-TCR.** A) Expression of the ckit marker (top) and the Notch1 oncogene (ICN1) (bottom) in bone marrow progenitor cells of normal mice (wt for wild-type) or mutant mice (C80G) transduced with a lentiviral vector carrying ICN1 and GFP. B) Percentages and absolute numbers of wt or C80G ICN1+ cells grafted in the bone marrow, spleen, blood, and liver of NSG mice at 5 weeks post-transplantation. C) Absolute numbers of ICN1+ and ICN1- cells grafted in the bone marrow of the mice in (B).

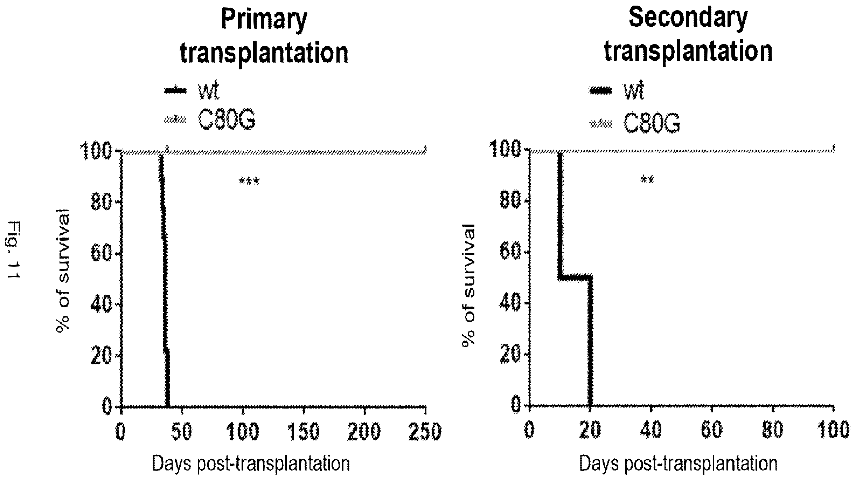

**Figure 11****. Survival of NSG mice transplanted with bone marrow progenitors with a normal or mutated pre-TCR function, transduced with ICN1.** Survival after a primary transplantation (left) or secondary transplantation (right) at the indicated post-transplantation days is shown.

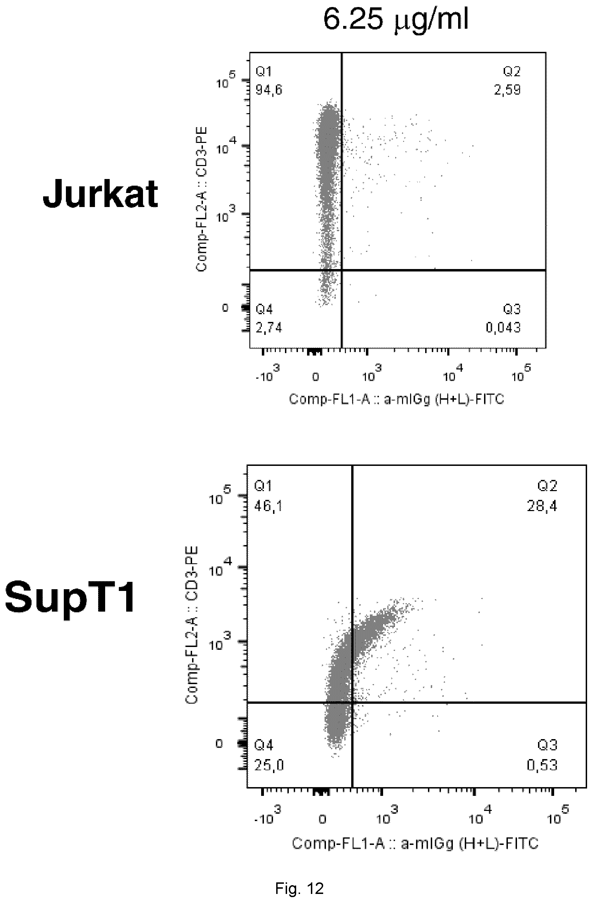

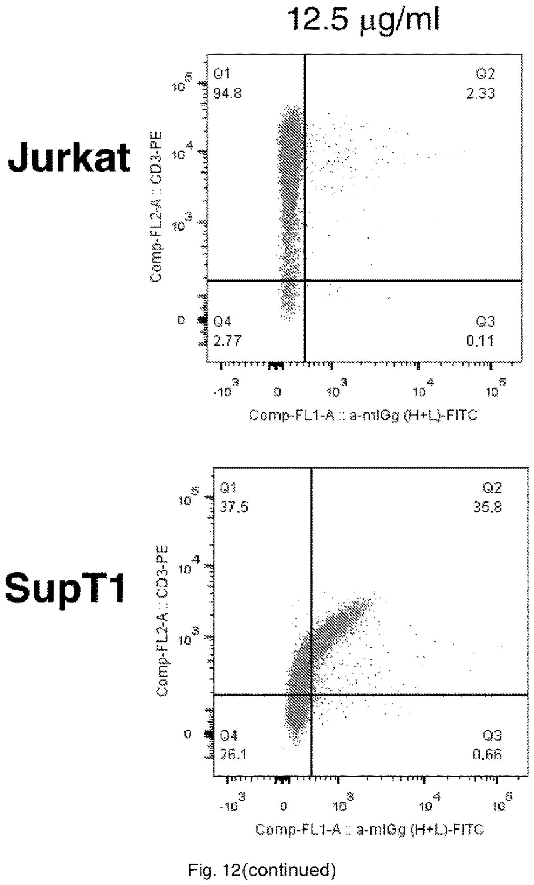

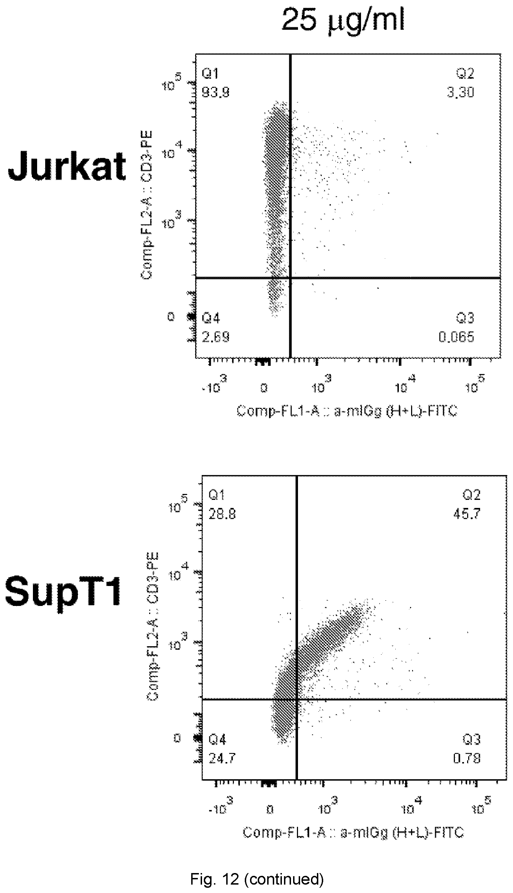

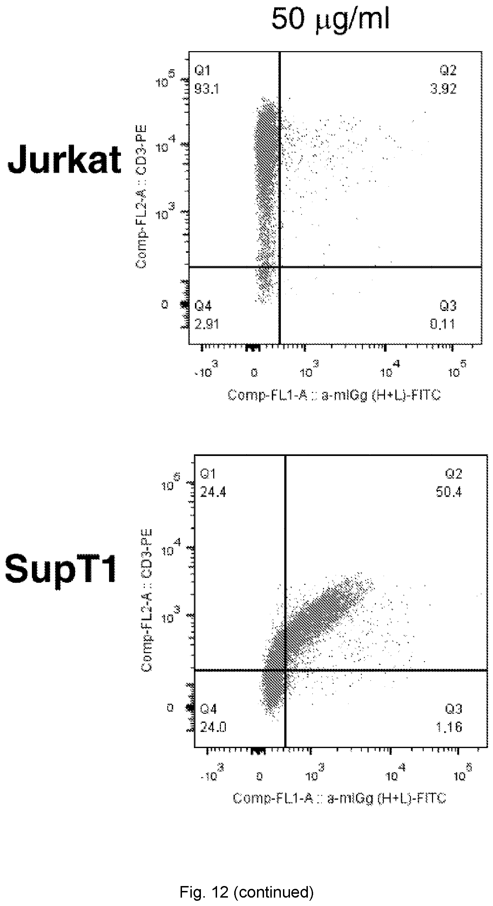

**Figure 12****. Titration of the antibody of the invention K5G3 (anti-pTα) in human T-ALL SUPT1 and Jurkat cell lines.** This figure shows the specific expression of pre-TCR in the SUPT1 line, measured by means of flow cytometry using anti-CD3ε antibody, UCHT1 (BD Biosciences), and increasing concentrations of the anti-pTα. antibody of the invention K5G3. Unlike UCHT1, K5G3 does not bind to the T-ALL Jurkat cell line which lacks pre-TCR, but expresses CD3-associated TCRαβ.

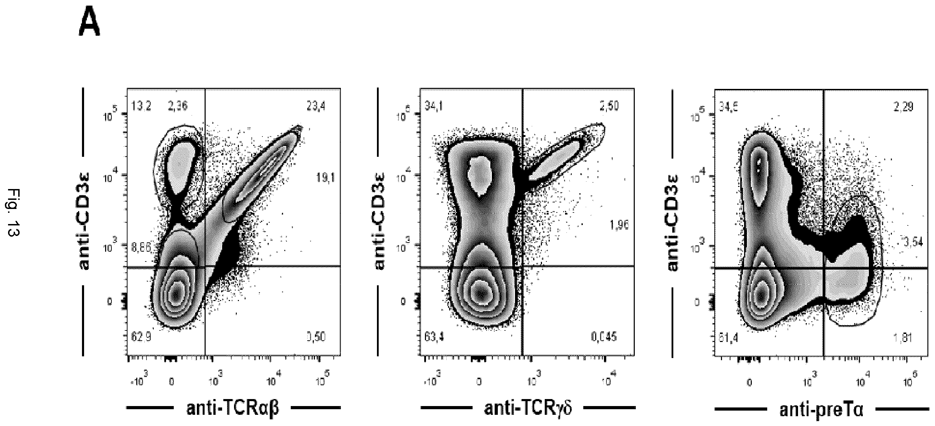

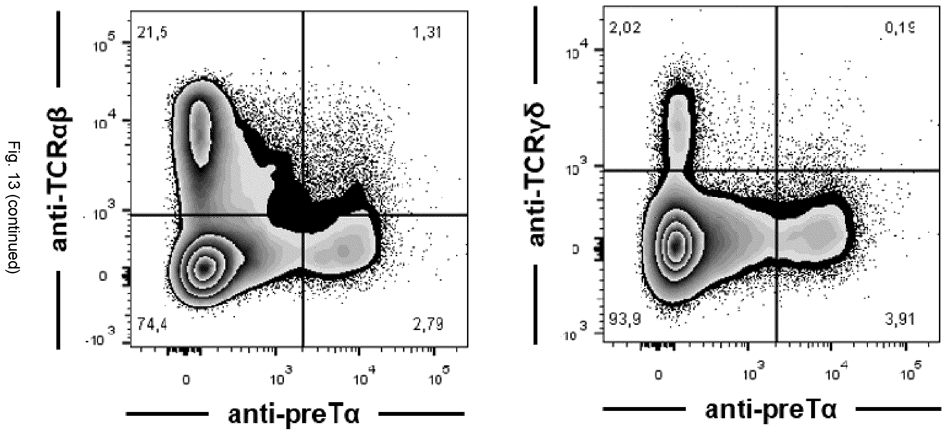

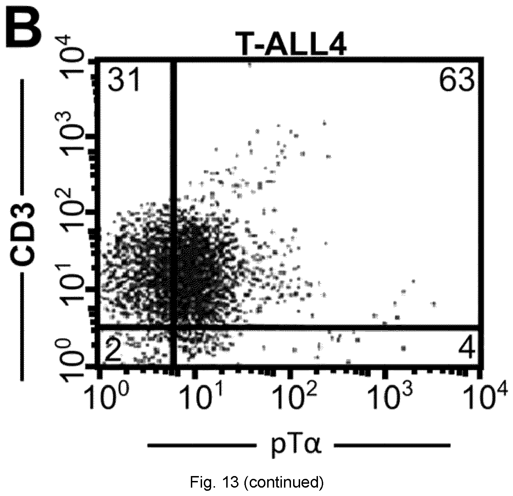

**Figure 13****. Binding of the antibody of the invention K5G3 (anti-pTα) to human thymic pre-T progenitors and to primary T-ALL cells of patients.** Analysis of the expression of pre-TCR using the anti-CD3ε antibody, UCHT1, and the antibody of the invention K5G3, anti-pTα, in (A) human thymus cells and (B) primary T-ALL cells obtained from a patient (T-ALL4). Phenotypic analyses in (A) show that subpopulations of human thymocytes expressing TCRαβ or TCRyδ, receptors in association with CD3, are not recognized by the anti-pTα, mAb, which demonstrates the specificity of the antibody of the invention K5G3.

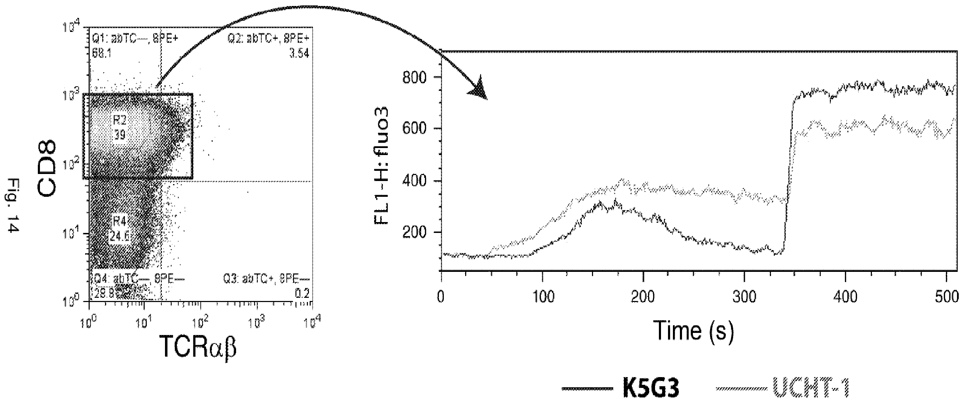

**Figure 14****. Study of the functionality of the human anti-pTα antibody of the invention, K5G3, in calcium mobilization assays.** Isolation of pre-TCR+ cells from human thymus by means of the immunomagnetic selection of CD8+ TCRαβ- thymocytes (left) and calcium mobilization in isolated pre-TCR+ thymocytes after the cross-linking of the pre-TCR with the anti-CD3ε antibody, UCHT1, or with the anti-pTα antibody of the invention, K5G3 (right).

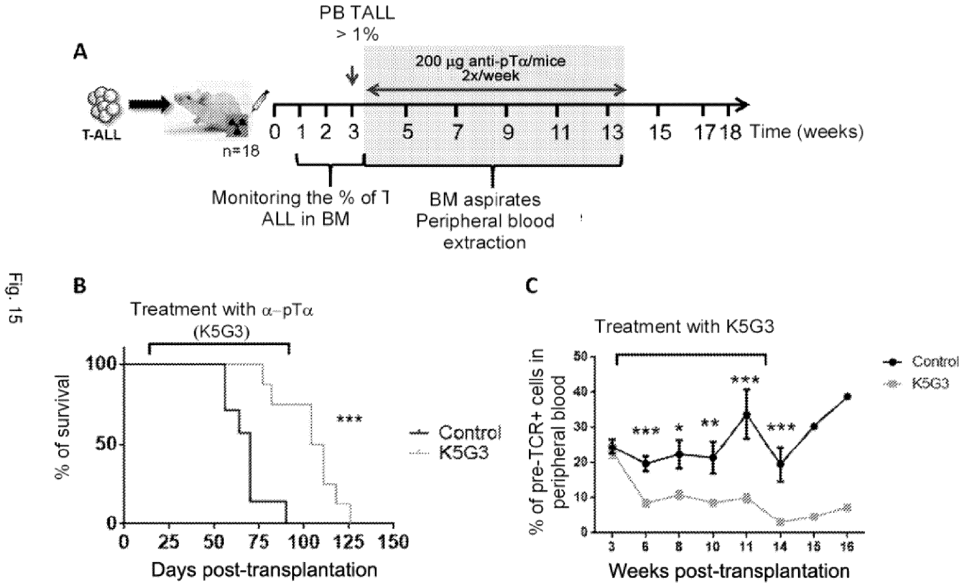

**Figure 15****. Immunotherapy by means of the administration of the anti-pTα antibody of the invention, K5G3, in a preclinical model of human T-ALL xenotransplantation.** A) Diagram of the dosage regimen of the anti-pTαantibody of the invention or of a control isotypic antibody (IgG2a), in SCID mice in which disease has been established following transplantation from a T-ALL patient. B) Survival of treated animals with respect to controls. C) Percentage of T-ALL pre-TCR+ cells in the peripheral blood (PB) of treated and control animals.

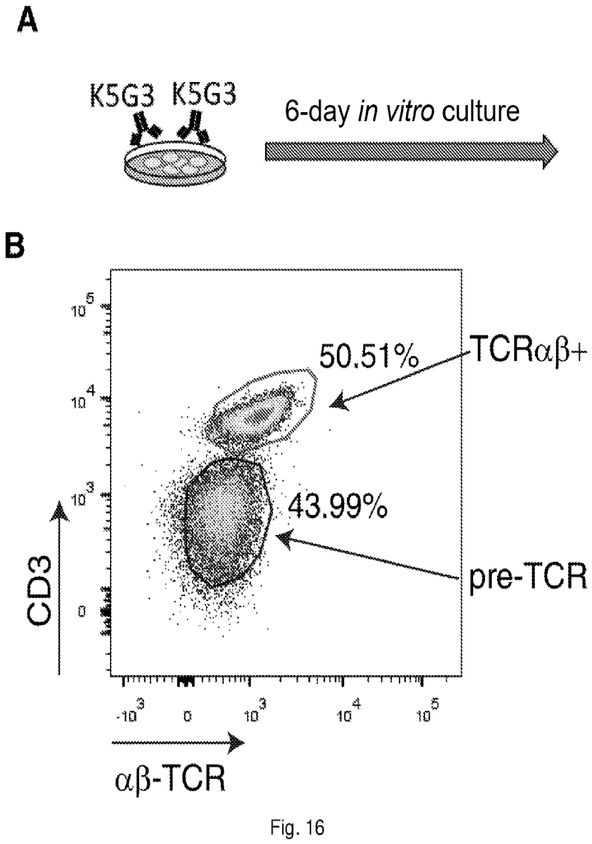

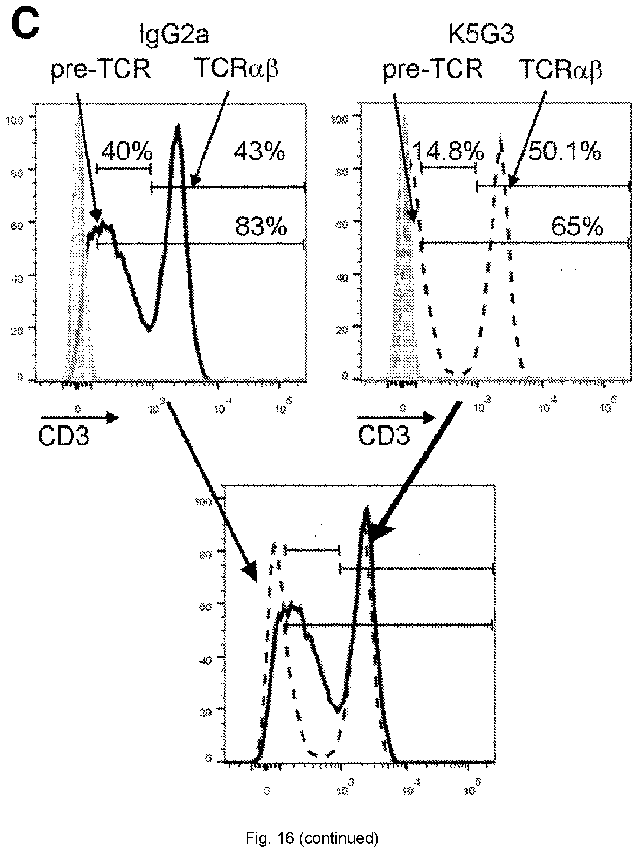

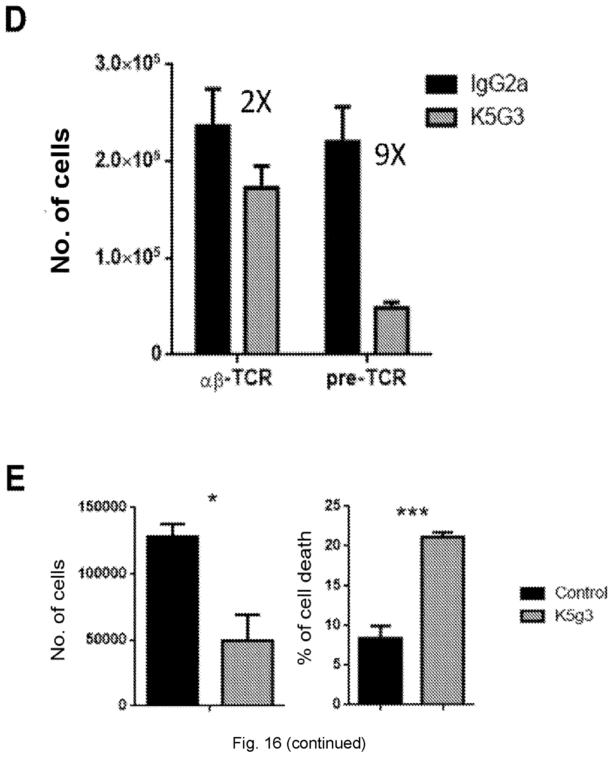

**Figure 16****. Effect of *in vitro* treatment with the anti-pTα antibody of the invention, K5G3, on the number of T-ALL pre-TCR+ cells.** A) Diagram of the *in vitro* culture of T-ALL in the presence of the anti-pTα antibody of the invention, K5G3, or of a non-specific antibody,lgG2a, used as a control. B) Heterogeneous TCRαβ+ (pre-TCR-) and pre-TCR+ (TCRαβ-) phenotype of the T-ALL leukemia cultured in (A). C) CD3ε expression levels in cells (B) after being cultured for 6 days in the presence of the anti-pTα antibody of the invention, K5G3, or of a non-specific antibody,lgG2a, used as a control. The superposition of both histograms where the selective reduction of pre-TCR+ cells is observed is shown below. D) Absolute numbers of TCRαβ+ and pre-TCR+ cells of the T-ALL leukemia in (B) after being cultured *in vitro* for 6 days in the presence of the anti-pTα antibody of the invention, K5G3, or of a control lgG2a. E) Absolute numbers and percentages of apoptotic cells recovered 6 days after culturing the T-ALL pre-TCR+ leukemia shown in Figure 5A in the presence of the anti-pTα antibody of the invention, K5G3, or of a control IgG2a. Data ± SEM derived from 3 experiments are shown.

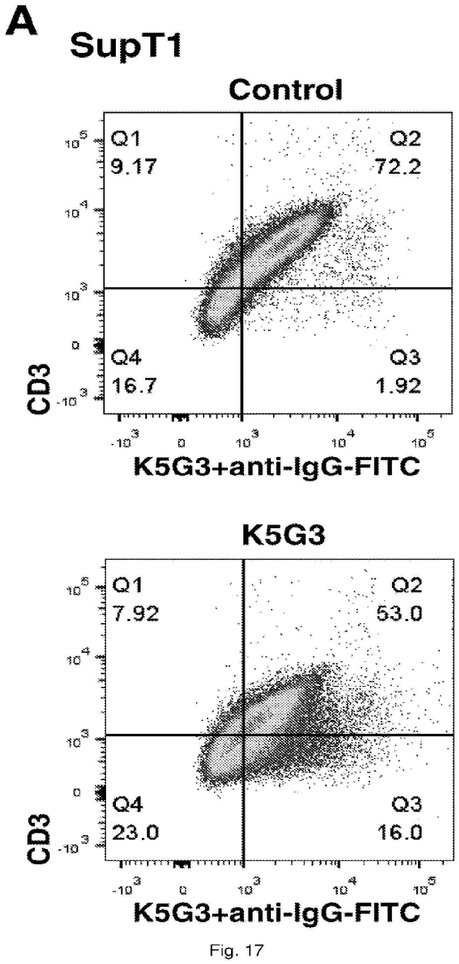

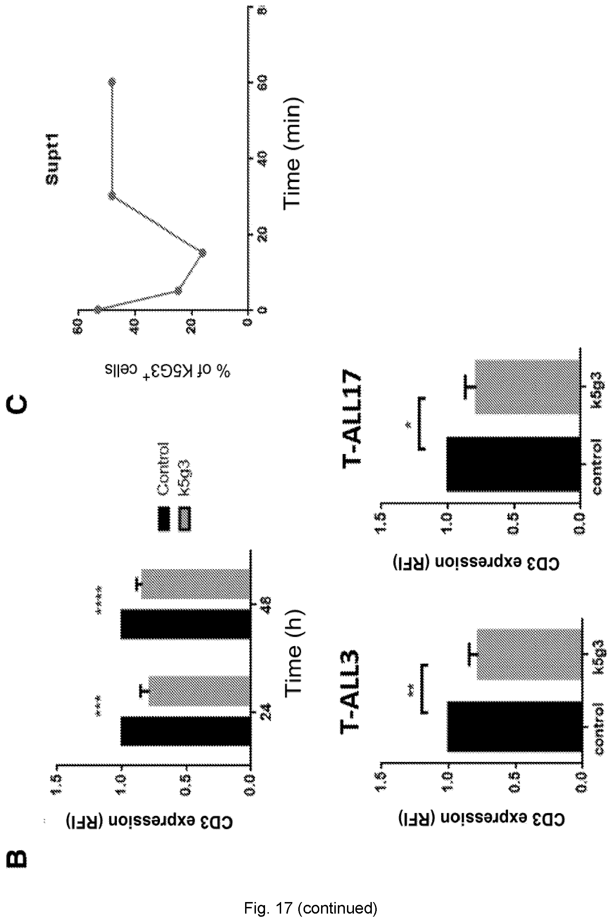

**Figure 17****. Effect of *in vitro* treatment with the human anti-pTα antibody of the invention, K5G3, on the internalization of pre-TCR in the SUPT1 line and in primary leukemias of patients.** A) Flow cytometry of the expression levels of CD3ε and pTα components of pre-TCR in SUPT1 cells analyzed after 24 hours of *in vitro* incubation with an irrelevant mAb IgG2a (control) or with the anti-pTα mAb of the invention (K5G3). B) CD3ε expression levels representative of pre-TCR levels in SUPT1 cells (top) or in primary T-ALL leukemias of patients (T-ALL3, T-ALL17) treated as described in (A). The data shows relative fluorescence intensity (RFI) values ± SEM of 3 experiments. C) Kinetics of the endocytosis and recycling of the CD3ε chain of the pre-TCR complex in SUPT1 cells treated for 30 min at 4°C (time=0) with an irrelevant mAb IgG2a (control) or with the anti-pTα mAb of the invention (K5G3), and then cultured at 37°C at the indicated times.

### EXAMPLES

Next, the invention will be illustrated by means of assays carried out by the inventors which clearly show the effectiveness of the antibody of the invention for the proposed medical uses and the fact that the receptor which recognizes the antibody of the invention, i.e., the pre-TCR receptor, is a suitable therapeutic target to combat relapses in T-ALL patients.

### Example 1. Generation of human T-ALL leukemias in NSG mice by means of the transplantation of umbilical cord blood HPCs overexpressing active Notch1 (ICN1).

In collaboration with the Transfusion Center of the Community of Madrid, umbilical cord blood samples have been obtained, from which hematopoietic progenitors (HPCs) were isolated by means of immunomagnetic selection with anti-CD34 antibodies. Selected cells were transplanted into NSG immunodeficient mice, after transduction thereof with a retroviral vector carrying the oncogenic form of Notch1 (ICN1) and GFP, as recently described (García-Peydró et al., J Clin Invest. 2 July 2018; 128(7): 2802-2818; Figure 1). In this model, the presence of human ICN1+ leukemic cells in peripheral blood and bone marrow aspirates from transplanted animals was analyzed over 8 months, which allowed identifying the *in vivo* generation of leukemia with characteristics of human T-ALL from 14-18 weeks in the bone marrow and from 30-36 weeks post-transplantation in the blood of transplanted mice (Figure 1). This data demonstrates for the first time the *de novo* generation of a human T-ALL leukemia *in vivo* from healthy cells.

### Example 2. Identification and in vivo monitoring of the expression of pre-TCR during the progression of human T-ALL in transplanted animals.

In order to monitor the cell populations involved in the pathogenesis of human T-ALL and the molecules involved, the phenotype of cells carrying the ICN1 oncogene (ICN1+) in bone marrow aspirates was analyzed by means of flow cytometry as indicated in Figure 2. The results obtained show that the pre-TCR complex is expressed early in the generation of leukemia and its expression on the surface of leukemic cells is maintained during the 10 months of T-ALL progression in transplanted animals. Likewise, the proportion of pre-TCR+ cells increases over time (Figure 2), which indicates that during the T-ALL generation process, the differentiation of T-lymphocytes in the maturation stage in which pre-TCR is expressed, expanding the pre-TCR+ population, is blocked, which suggests a relevant role of this receptor in the progression of the disease. To establish the optimal conditions for the *in vivo* monitoring of the progression of pre-TCR+ human leukemias, a xenogeneic transplantation model was developed in NSG immunodeficient mice using, as a control of T-ALL pre-TCR+ cells, the SUPT1 cell line which co-expresses pre-TCR CD3ε and pTα molecules which are recognized by the mAb, UCHT1 (BD Biosciences), and the antibody of the invention (K5G3), respectively; meanwhile TCR+ leukemias, such as HPB-ALL, only express CD3ε (Figure 3). The model was extended to the analysis of primary T-ALL pre-TCR+ leukemias from patients. The cells were transduced with a bicistronic lentiviral vector (pHRsin) carrying the GFP and luciferase gene, which makes it possible to follow the progression of leukemia and the infiltration of different peripheral organs in the transplanted mice. By means of this experimental strategy, it was possible to demonstrate the efficient *in* vivoexpansion of SUPT1 pre-TCR+ leukemia and primary T-ALL leukemias (Figure 3).

### Example 3. In vivo validation and quantification of leukemia-initiating activity (LIC) of human pre-TCR+ leukemic cells generated de novo in animals and of primary T-ALL leukemias from patients.

To check the relevance of the pre-TCR complex in the *in vivo* expansion of T-ALL, serial transplantations of the leukemic cells generated in mice transplanted with ICN1-transduced human HPC progenitors were performed (Figure 1). Cells from the bone marrow of these animals, showing a majority CD4+CD8+ DP phenotype and containing a significant pre-TCR+ subpopulation (35%), were transplanted into a second 6-8 week old NSG recipient mouse, sublethally irradiated with 1.5 Gy, and so on and so forth up to 3 transplantations. This assay allowed the LIC activity of pre-TCR+ cells in successive transplantations to be analyzed. Analysis of the phenotype of the T-ALL cells obtained from the bone marrow after 3 transplantations showed pre-TCR expression in the membrane of >95% of the leukemic cells that were expanded in the transplanted animals (Figure 4), which demonstrates an *in vivo* proliferative advantage of pre-TCR+ cells over pre-TCR- cells. Subsequent biochemical analyses of the expanded cells corroborated the function of the pre-TCR complex in the activation of molecular pathways involved in cell survival and proliferation, in response to stimulation induced with anti-pre-TCR monoclonal antibodies (anti-CD3ε or the anti-pre-Tα antibody of the invention K5G3). These pathways include: Pi3K/Akt, Erk, y mTORC1/S6 (Figure 5). Therefore, the LICs of the T-ALLs generated express a functional pre-TCR involved in cell proliferation. Similar experiments were performed with peripheral blood primary leukemia cells from patients diagnosed with T-ALL (T-ALL17), using the mouse xenotransplantation model. Human T-ALL cells were obtained by Ficoll-Hypaque centrifugation and injected (10⁵) intravenously into 6-8 week old NSG mice, sublethally irradiated with 1.5 Gy. The *in vivo* expansion potential of the primary leukemic cells was analyzed by means of flow cytometry performed on bone marrow aspirates from mice at different weeks post-transplantation. The study revealed that serial transplantations of T-ALL samples expressing very low levels of CD3 induce sequential enrichment in these cells, comprising the pre-TCR+ population, which demonstrates its *in vivo* proliferation advantage and its LIC activity. Biochemical assays subsequently corroborated the functionality of pre-TCR inducing the activation of survival and proliferation pathways also in primary T-ALLs (Figure 6). Overall, this data demonstrates that human T-ALL pre-TCR+ cells exhibit LIC activity, and therefore confirms that pre-TCR can be a therapeutic target to combat relapses in patients.

To confirm that pre-TCR is a biomarker for cells with LIC activity, involved in the progression of leukemia-initiating cells, the two pre-TCR+ and pre-TCR- (TCRαβ+) subpopulations included in T-ALL17 leukemia were separated and the LIC activity of both subpopulations was analyzed separately in xenotransplantations with decreasing cell dilutions, according to the diagram shown in Figure 7.

Next, the frequency of cells with LIC activity in the pre-TCR+ and pre-TCR- populations of the same patient was analyzed by means of monitoring the survival of transplanted animals by Kaplan-Meier estimation, using the ELDA program (http://bioinf.wehi.edu.au/software/elda/). The results showed that LIC cells are enriched about 40-fold in the pre-TCR+ population compared to the pre-TCR- population. Accordingly, mice transplanted with pre-TCR+ cells showed significantly decreased survival compared to mice receiving pre-TCR- cells from the same patient (Figure 8).

### Example 4. Analysis of the function of pre-TCR in T-ALL LIC activity: CMS adapter silencing in human T-ALL leukemias and analysis of the L!C activity thereof in in vivo preclinical models.

To directly determine the relevance of pre-TCR in T-ALL LIC activity, and therefore in the progression and relapse of T-ALL in patients, the impact of inhibition of pre-TCR function on the tumor progression of human T-ALL cells transplanted in NSG immunodeficient mice was analyzed. To that end, previous results published by the inventors (Navarro et al. Blood. 15 December 2007;110(13):4331-40), indicating that the function of pre-TCR is dependent on the CMS/CD2AP adapter that binds to pTα, were considered, and strategies involving CMS gene silencing mediated by short-hairpin(sh)RNA-CMS expressed in bicistronic lentiviral vectors also carrying GFP, or vectors carrying a control shRNA (shsc) were used (Figure 9). Optimal silencing conditions were established using the T-ALL pre-TCR+ SUPT1 line, that were transferred to two primary leukemias (T-ALL5 and T-ALL9) from patients, The transduction efficiency of which was analyzed by means of GFP expression (Figure 9). Modified T-ALL was transplanted intravenously and leukemic progression was analyzed in the transplanted animals, with a drastic inhibition of tumor progression being observed after CMS silencing (Figure 9), which translated into a complete blockade of the induction of splenomegaly observed in animals transplanted with T-ALLs transduced with control vectors (Figure 9). Therefore, intact pre-TCR signaling is required for the *in vivo* progression of human T-ALL cells, demonstrating that the inhibition of the function of the pre-TCR expressed in T-ALL cells efficiently blocks their LIC activity and their *in vivo* expansion, which indicates that the pre-TCR is a therapeutic target of T-ALL.

### Example 5. Analysis of the relevance of pre-TCR as a molecular target of cells with LIC activity involved in the generation of human T-ALL.

Phenotypic analysis of leukemic cells generated in the human T-ALL leukemia induction model (Figure 1) showed the majority expression of pre-TCR in all the generated leukemias (Figure 2). More importantly, serial transplantations suggested an *in vivo* proliferative advantage of pre-TCR+ cells over pre-TCR- cells and the involvement of pre-TCR in the function of cells with LIC activity involved in the generation of T-ALL (Figure 4).

In order to confirm the functional involvement of pre-TCR in the generation of T-ALL, the tumor potential of the Notch1 oncogene was subsequently examined in hematopoietic progenitors from the bone marrow of normal mice (wt for wild-type), or mice with a mutation (C80G) which prevents pre-TCR-mediated signaling (White et al. Science Signal. 2 December 2014;7(354):ra115). Progenitors from the bone marrow of normal or C80G mice transduced with an oncogenic form of GFP-associated Notch1 (ICN1) were transplanted into NSG immunodeficient mice, and the generation of T-ALL leukemia was analyzed at different times in the transplanted animals by monitoring GFP+ tumor cells in different organs.

Flow cytometric analysis demonstrated a similar expression of the ICN1 oncogene in normal and C80G cells. However, only normal cells showed the capacity of grafting and expanding in the bone marrow, blood, spleen, and other organs, such as the liver, in the transplanted animals 5 weeks post-transplantation, whereas mutant cells were not capable of generating leukemia in any of the organs analyzed at different times (Figure 10). Accordingly, the survival of animals transplanted with mutant cells carrying the oncogene was not affected, with the survival of animals transplanted with normal cells, as well as the survival of second recipients transplanted with these cells, being dramatically reduced (Figure 11). Therefore, it can be concluded that the function of pre-TCR is essential in the generation of T-ALL leukemia induced by the ICN1 oncogene, which demonstrates that the pre-TCR complex is an optimal target for therapeutic intervention.

### Example 6. Analysis of the prevalence of pre-TCR expression in human primary T-ALLs.

The data obtained in Example 4 and Example 5 constitute proof of concept of the suitability of a new therapeutic strategy aimed at eliminating the function of pre-TCR and/or of leukemic cells carrying same. Therefore, the next objective was to evaluate the prevalence of pre-TCR expression in T-ALLs of patients and to determine the frequency of patients carrying pre-TCR+ LIC, who are potential beneficiaries of the proposed treatment. To that end, collaboration was established with the SEHOP Acute Leukemia Group, specifically, with Dr. Manuel Ramirez Orellana from Hospital Infantil Niño Jesús in Madrid and with Dr. Mireia Camós from Hospital Sant Joan de Déu in Barcelona, as well as with Dr. Antonio Pérez Rodriguez from Hospital Universitario La Paz in Madrid, to determine pre-TCR expression in patients at diagnosis by means of flow cytometry. The samples were analyzed with the antibody of the invention. 13 T-ALL samples were analyzed, with pre-TCR expression being observed in 7 of them, which constitutes >50% of the samples analyzed (Table 1). Therefore, a significant number of patients with T-ALL could benefit from the proposed anti-pre-TCR treatment.

**Table 1: Prevalence of pre-TCR expression in patients diagnosed with T-ALL**

| Samples (a) | TCR | PRE-TCR | IL7R | CXCR4 |
|---|---|---|---|---|
| T-ALL1 | + | - | - | ++ |
| T-ALL2 | + | - | ++ | ND |
| T-ALL3 | - | + | ++ | ND |
| T-ALL5 | - | + | + | ++ |
| T-ALL8 | + | - | + | ++ |
| T-ALL9 | + | - | + | ++ |
| T-ALL10 | + | - | + | ++ |
| T-ALL17 | + | + | + | ++ |
| T-ALL18 | + | + | ++ | ++ |
| T-ALL26 | - | + | ++ | ++ |
| T-ALL29 | + | + | ND | ND |
| T-ALL30 | - | + | ++ | ++ |
| T-ALL32 | ++ | - | ++ | ND |

| | | | | |
|---|---|---|---|---|
| (a) The expression of pre-TCR, TCR, IL-7 receptor (IL-7R), and chemokine receptor CXCR4 in the indicated primary samples, obtained from the peripheral blood or bone marrow of patients diagnosed with T-ALL, was analyzed. | | | | |

### Example 7. Study of the functional characteristics of the antibody of the invention. Production and purification.

The antibody of the invention is a mouse IgG2a monoclonal antibody directed against the extracellular domain of human pTa, the specific component of pre-TCR which associates with the TCRβ chain and the CD3 complex. The antibody was produced as follows: the hybridoma, K5G3, deposited in the European Collection of Cell Cultures under accession number 01080805, was expanded in large quantities using a hollow fiber bioreactor (Fiber Cell Systems). The secreted monoclonal antibody was purified from the culture supernatant by means of affinity chromatography on a protein G Sepharose column (GE Healthcare), and the purified monoclonal antibody was subsequently analyzed in functional *in vitro* assays. The studies indicate that the antibody of the invention is capable of binding specifically to the pre-TCR expressed in the SUPT1 cell line in a concentration-dependent manner, but not to the TCR complex formed by a TCRα chain attached to the TCRβ chain and the CD3 complex in the T-ALL Jurkat cell line (Figure 12). Despite low levels of pre-TCR expression in human pre-T cells, the monoclonal antibody of the invention is also capable of binding specifically to the pre-TCR expressed in human pre-T thymocytes, without binding to TCRαβ or TCRγδ cells of the thymus (Figure 13), and inducing the activation of these thymocytes mediated by calcium mobilization, at levels similar to those induced by the anti-CD3ε monoclonal antibody UCHT1 (Figure 14). Furthermore, as shown in Figures 5 and 6, the antibody of the invention is as effective as UCHT1 (or even more so) in inducing the activation of PI3K/AKT/mTOR and MAPK signaling pathways in primary human T-ALL pre-TCR+ cells.

### Example 8. In vivo validation of the efficacy of an immunotherapy targeting T-ALL pre-TCR+ LICs in preclinical models.

The success of treatments such as RITUXIMAB, based on the principle of specific recognition of a membrane protein of leukemia (B-ALL in this case), and the subsequent elimination of cells carrying the protein by a monoclonal antibody, suggests that the strategy of this invention may be valid. The fact that the chosen molecule, the pTα chain of pre-TCR, is selectively expressed during intrathymic development but absent in peripheral T-lymphocytes, represents an additional value of the strategy for preventing adverse effects due to the elimination of normal mature T-cells which could induce an aggressive immunodeficiency.

To analyze the efficiency of the proposed immunotherapy *in vivo,* SCID mice irradiated with sublethal doses (1.5 Gy) and transplanted with primary T-ALL cells from peripheral blood waste samples of T-ALL patients at diagnosis were used (Figure 15). The SCID strain, unlike NSG, has intact complement-dependent cytotoxicity (CDC) function, as well as antibody-dependent cytotoxicity (ADCC) function. Animals transplanted with T-ALL were examined on different days post-transplantation and the presence of human leukemic cells in peripheral blood (PB) was determined. The disease was considered to be established when the animals presented >1% of leukemic cells in blood (>3 weeks post-transplantation). At this point, Treatment, consisting of the intraperitoneal administration of the antibody of the invention or of a isotypic control monoclonal antibody (200 µg/mouse), 2 times a week for 10 weeks, was started (Figure 15). Throughout treatment, the graft in bone marrow aspirates, as well as infiltration into different lymphoid organs (thymus, spleen, and blood) and non-lymphoid organs (liver and brain) in the transplanted animals, were analyzed, observing a selective decrease in leukemic cells in animals treated with the antibody of the invention in various organs. Although treatment discontinuation resulted in leukemia progression, it should be noted that the survival of the treated animals was significantly greater than the survival of the control animals (from 70 to 110 days on average) (Figure 15), obtaining similar results with another leukemia from a patient diagnosed with T-ALL, which validates the efficiency of the proposed immunotherapy.

The phenotypic analysis of the organs of the treated animals showed a selective effect on leukemic cells carrying pre-TCR, which decreased compared to pre-TCR- cells, which ensures the specificity of the treatment (Figure 15). Additional preliminary analyses suggest that the antibody of the invention induces cell death by *in vivo* apoptosis of tumor cells through a CDC and/or ADCC induction mechanism.

### Example 9. Study of the capacity of the anti-pTα antibody of the invention, K5G3, to induce pre-TCR endocytosis and apoptosis in pre-TCR+ cells.

*In vitro* studies using heterogeneous primary T-ALL cells for pre-TCR and TCRαβ, expression which are cultured for several days in the presence of the anti-pTα antibody of the invention or an irrelevant antibody of the same isotype (IgG2a) indicate that the antibody of the invention not only induces the activation of pre-TCR+ leukemic cells (Figure 5, Figure 6), but also induces their specific disappearance, which correlates with the induction of cell death by apoptosis (Figure 16).

Furthermore, *in vitro* incubation studies of SUPT1 pre-TCR+ cells with the anti-pTα antibody of the invention for short periods of time had demonstrated that this antibody also induces the internalization of pre-TCR from the cell surface (Navarro et al. Blood. 15 December 2007;110(13):4331-40). The results shown in Figure 17 corroborate this data in SUPT1 cells and extend said data to primary T-ALL pre-TCR+ leukemias obtained from patients, with a significant decrease in expression levels, and therefore of the percentage of pre-TCR+ cells, being detected in both cases after short incubation periods. Furthermore, endocytosis kinetics studies by means of flow cytometry corroborated the internalization capacity of the anti-pTα antibody of the invention within a few minutes of binding to its target, i.e., pre-TCR, in the cell membrane (Figure 17), so it is a molecular mechanism which can be exploited from a therapeutic viewpoint, since therapies based on the use of antibodies conjugated to toxins/drugs (ADC for antibody-drug conjugates) are particularly effective in inducing cell lysis in cases where the antibody is internalized together with the corresponding molecular targets.

## Claims

1. A monoclonal antibody obtained by the hybridoma deposited with the European Collection of Cell Cultures under accession number 01080805, capable of specifically recognizing the TCRβ- and CD3-associated pTα chain of the pre-T cell receptor (pre-TCR), for use as a medicament.

2. The antibody according to claim 1, for use in the treatment and/or prevention of leukemia, preferably T-cell acute lymphoblastic leukemia (T-ALL), more preferably T-ALL pre-TCR+.

3. The antibody according to any one of claims 1 to 2, for use in the treatment and/or prevention of relapses of leukemia, preferably relapses of T-cell acute lymphoblastic leukemia (T-ALL), more preferably relapses of T-ALL pre-TCR+.

4. The antibody according to any one of claims 1 to 3, wherein said antibody is coupled to a toxin.

5. The antibody according to any one of claims 1 to 4, wherein said antibody is humanized.

6. A pharmaceutical composition comprising the monoclonal antibody obtained by the hybridoma deposited with the European Collection of Cell Cultures under accession number 01080805, capable of specifically recognizing the TCRβ- and CD3-associated pTα chain of the pre-T cell receptor (pre-TCR), for use in the treatment and/or prevention of leukemia, preferably T-cell acute lymphoblastic leukemia (T-ALL), more preferably T-ALL pre-TCR+.

7. The pharmaceutical composition according to claim 6, for use in the treatment and/or prevention of relapses of leukemia, preferably relapses of T-cell acute lymphoblastic leukemia (T-ALL), more preferably relapses of T-ALL pre-TCR+.

8. The pharmaceutical composition according to any one of claims 6 or 7, wherein the antibody is coupled to a toxin.

9. The pharmaceutical composition according to any one of claims 6 to 8, wherein the antibody is humanized.

10. The pharmaceutical composition according to any one of claims 6 to 9, further comprising an acceptable pharmaceutical adjuvant, an acceptable pharmaceutical carrier, and/or another active ingredient.
